# EUROPEAN PATENT APPLICATION

(11) **EP 4 230 643 A1**
(43) Date of publication of application: **23.08.2023**
(21) Application number: 21880221.3
(22) Date of filing: 15.10.2021
(51) Int. Cl.: C07K 14/36, C07K 17/00, C12N 15/31, C12Q 1/00, C12Q 1/68, G01N 33/53

(54) **POLYPEPTIDE, POLYMER, SOLID PHASE, MEASUREMENT METHOD FOR TEST SUBSTANCE, AND REAGENT KIT**

(30) Priority: 16.10.2020 JP 2020174961
(71) Applicant: SYSMEX CORPORATION, Kobe-shi Hyogo 651-0073 (JP)
(72) Inventor: SUGANUMA, Masatoshi, Kobe-shi, Hyogo 651-0073 (JP); NISHIKAWA, Youichi, Kobe-shi, Hyogo 651-0073 (JP); KUBO, Takuya, Kobe-shi, Hyogo 651-0073 (JP)
(74) Representative: De Clercq & Partners
(86) International application number: PCT/JP2021/038254
(87) International publication number: WO 2022/080486

(57) **Abstract**

An object is to provide a polypeptide that further reduces binding to D-biotin and strongly binds to L-biotin. Provided is a polypeptide belonging to the avidin-streptavidin family, in which the polypeptide contains a D-amino acid residue in 90% or more of amino acid residues among amino acid residues other than glycine of the polypeptide, has a binding ability to L-biotin, and has substantially no binding ability to D-biotin.

## Description

### TECHNICAL FIELD

The present invention relates to a polypeptide, a multimer, a solid phase, and a measurement method for a test substance, and a reagent kit.

### BACKGROUND ART

The affinity between a polypeptide belonging to the avidin-streptavidin family and biotin is very strong, and the polypeptide and biotin are utilized in various fields. For example, in the field of diagnosis, in an immunological measurement method, a capture body is immobilized on a solid phase such as a plate or a particle via streptavidin and biotin, and a target substance is measured using the capture body. In addition, in the therapeutic field, a pre-targeting therapy in which an antibody to which avidin is added and a drug to which a biotin group is added are administered to a patient is known (for example, Non Patent Document 1).

However, when blood of a subject contains natural D-biotin, this D-biotin (endogenous D-biotin) may bind to avidin or streptavidin. In particular, when the subject takes a supplement such as a vitamin, the blood concentration of D-biotin may be high. In such a case, in an immunological measurement method using a blood specimen, a biotinylated capture body and an endogenous D-biotin in the blood compete with each other, and a part of the capture body cannot be immobilized on the solid phase, and the measurement accuracy may be reduced. Also, in the pre-targeting therapy, when an antibody to which avidin is bound is administered to a patient, endogenous D-biotin in the blood may be bound to avidin. In this case, a drug in which a biotin group is added to the antibody cannot be bound, and there may be a problem that the amount of the drug reaching the affected area is reduced. As a means for solving such a problem, for example, the technology of Patent Document 1 is known. Patent Document 1 discloses a streptavidin mutant with reduced affinity for D-biotin and a modified biotin capable of binding to the mutant.

### PRIOR ART DOCUMENTS

### PATENT DOCUMENT

Patent Document 1: US 2016/0,137,704

### NON PATENT DOCUMENT

Non Patent Document 1: Hnatowich et al., Journal of Nuclear Medicine 28.8 (1987): 1294-1302

### SUMMARY OF THE INVENTION

### PROBLEMS TO BE SOLVED BY THE INVENTION

The streptavidin mutant described in Patent Document 1 still maintains binding to D-biotin, and the affinity between the mutant and a biotin variant is not sufficient. An object of the present invention is to provide a polypeptide that does not substantially bind to D-biotin and strongly binds to L-biotin. Also, an object of the present invention is to provide a reagent kit including a multimer of the polypeptide, a solid phase on which the polypeptide is immobilized, a measurement method for a test substance using the solid phase, and a capture body to which the solid phase and L-biotin are bound.

### SOLUTIONS TO THE PROBLEMS

Provided is a polypeptide belonging to the avidin-streptavidin family, in which the polypeptide contains a D-amino acid residue in 90% or more of amino acid residues among amino acid residues other than glycine of the polypeptide, has a binding ability to L-biotin, and has substantially no binding ability to D-biotin.

The present invention provides the polypeptide and a multimer of the polypeptide. The present invention provides a reagent kit including a solid phase on which the polypeptide and the multimer are immobilized, a measurement method using the solid phase, and a capture body to which the solid phase and L-biotin are bound.

### EFFECTS OF THE INVENTION

According to the present invention, a polypeptide that does not substantially bind to D-biotin and has binding ability to L-biotin is provided. The present invention also provides a reagent kit including a multimer of the polypeptide, a solid phase to which the polypeptide is bound, a measurement method using the solid phase, and a capture body to which the solid phase and L-biotin are bound.

### BRIEF DESCRIPTION OF THE DRAWINGS

Fig. 1 is a schematic diagram showing an example of the measurement method of the present invention.
Fig. 2 is a schematic diagram showing an example of the reagent kit of the present invention.
Fig. 3 is a graph showing a change in absorbance at a wavelength of 280 nm in gel filtration chromatography.
Fig. 4A is a sensorgram of surface plasmon resonance (SPR) of the polypeptide of Example 2 and L-biotin.
Fig. 4B is a sensorgram of surface plasmon resonance (SPR) of the polypeptide of Example 2 and D-biotin.
Fig. 5 is a sensorgram of surface plasmon resonance (SPR) of natural core streptavidin of Example 2 and D-biotin-labeled albumin.
Fig. 6 is a graph showing the results of Example 3.
Fig. 7 is a graph showing the results of Example 4.
Fig. 8A is a structural model before substituting the 21st amino acid related to structure maintenance in Example 5 with an L-form.
Fig. 8B is a structural model after substituting the 21st amino acid related to structure maintenance in Example 5 with an L-form.
Fig. 8C is a structural model obtained by substituting the 21st amino acid related to structure maintenance in Example 5 with an L-form and then performing structure optimization by Clean Geometry.
Fig. 9A is a sensorgram of surface plasmon resonance (SPR) of D-tamavidin (trademark) 2 and L-biotin-labeled albumin in Example 7.
Fig. 9B is a sensorgram of surface plasmon resonance (SPR) of D-tamavidin (trademark) 2 and D-biotin-labeled albumin in Example 7.
Fig. 10 is a graph showing the results of Example 8.
Fig. 11 is a graph showing the results of Example 9.
Fig. 12A is a structure of L-biotin.
Fig. 12B is a structure of D-biotin.

### MODE FOR CARRYING OUT THE INVENTION

### 1. Polypeptide

The polypeptide of the present invention belongs to the avidin-streptavidin family. In this polypeptide, 90% or more of amino acid residues among amino acid residues other than glycine are D-amino acid residues. This polypeptide is a polypeptide that has binding ability to L-biotin and substantially no binding ability to D-biotin.

Polypeptides of the avidin-streptavidin family are composed of L-amino acid residues in nature, except for glycine, where no optical isomer exists. The polypeptide of the present invention may contain an L-amino acid residue, but 90% or more of amino acid residues among amino acid residues other than glycine are preferably D-amino acid residues. In the polypeptide of a more preferred embodiment, 95% or more of amino acid residues among amino acid residues other than glycine are D-amino acid residues, and in the polypeptide of a further preferred embodiment, all amino acid residues other than glycine are D-amino acid residues. Thus, it is considered that at least a steric structure of a site that binds to D-biotin becomes an enantiomer. As a result, the binding to a natural form of D-biotin is reduced, and it becomes possible to bind to L-biotin which is an enantiomer of D-biotin.

The polypeptide of the present invention does not substantially bind to D-biotin even when D-biotin is present in the blood of a subject, and thus can reduce the influence of endogenous D-biotin in an immunological measurement method. In addition, in a pre-targeting therapy, the influence of endogenous D-biotin is reduced, and the drug can be more appropriately delivered to the affected area.

As used herein, the "polypeptide" includes a protein and its fragments. The length of amino acids of the polypeptide of the present invention is not particularly limited as long as it has binding ability with L-biotin, and is, for example, several tens to several hundreds of residues. The polypeptide of the present invention preferably has at least a core sequence. Here, the "core sequence" refers to an amino acid sequence necessary for binding to biotin among the amino acid sequences of the polypeptide of the present invention.

The "polypeptides belonging to the avidin-streptavidin family" include avidin, streptavidin, avidin-like protein (hereinafter, tamavidin (trademark)) derived from Pleurotus cornucopiae, Bradavidin, Rhizavidin, chimeras and variants thereof, and the like. In the present specification, polypeptides of type D (for example, avidin of type D (hereinafter, referred to as "D-avidin" or "avidin of the present invention"), streptavidin of type D (hereinafter, referred to as "D-streptavidin" or "streptavidin of the present invention"), tamavidin (trademark) of type D (hereinafter, referred to as "D-tamavidin (trademark)" or "tamavidin (trademark) of the present invention"), bradavidin of type D (hereinafter referred to as "D-bradavidin"), and rhizavidin of type D are also included in the "polypeptides belonging to the avidin-streptavidin family".

Examples of the variants of polypeptide include polypeptides with a modified amino acid sequence, and polypeptides obtained by chemically treating the polypeptide. Examples of modification of the amino acid sequence include substitution, deletion, and addition of amino acid residues. Examples of chemical treatments include deglycosylation and the like.

Streptavidin is a biotin-binding protein derived from Streptomyces avidinii.

In a preferred embodiment, D-streptavidin contains the 19th to 133rd amino acid sequence of SEQ ID NO: 1. The 19th to 133rd amino acid sequence is known as a core sequence of D-streptavidin. In a more preferred embodiment, D-streptavidin contains the 13th to 133rd amino acid sequence or the 19th to 133rd amino acid sequence. In a further preferred embodiment, D-streptavidin contains the 13th to 139th amino acid sequence. In another embodiment, D-streptavidin contains an entire amino acid sequence of SEQ ID NO: 1. In the streptavidin of the present invention, since 90% or more of amino acid residues among amino acid residues other than glycine are composed of D-amino acid residues, the binding to D-biotin is low and the binding to L-biotin is high.

Avidin is a biotin-binding protein produced in birds and the like. D-avidin, which is an embodiment of the present invention, may include an amino acid sequence of SEQ ID NO: 2. In a preferred embodiment, D-avidin contains the 2nd to 128th amino acid sequence of SEQ ID NO: 2. The 2nd to 128th amino acid sequence is considered to be a core sequence of D-avidin. In another embodiment, D-avidin contains an entire amino acid sequence of SEQ ID NO: 2. In the avidin of the present invention, since 90% or more of amino acid residues among amino acid residues other than glycine are composed of D-amino acid residues, the binding to D-biotin is low and the binding to L-biotin is high.

Tamavidin (trademark) is a protein discovered from Pleurotus cornucopiae. It has characteristics such as high affinity for biotin and excellent thermal stability as compared with avidin (WO 2002/072817 A). Examples of the amino acid sequence of tamavidin (trademark) include the amino acid sequences shown in SEQ ID NO: 3 (tamavidin (trademark) 1) and SEQ ID NO: 4 (tamavidin (trademark) 2).

In a preferred embodiment, D-tamavidin (trademark) 1 contains the 4th to 129th amino acid sequence of SEQ ID NO: 3. The 4th to 129th amino acid sequence is considered as a core sequence of tamavidin (trademark) 1. D-Tamavidin (trademark) 2 contains the 4th to 127th amino acid sequence of SEQ ID NO: 4. The 4th to 127th amino acid sequence is considered as a core sequence of tamavidin (trademark) 2. In D-tamavidin (trademark) 1 and D-tamavidin (trademark) 2, since 90% or more of amino acid residues among amino acid residues other than glycine are composed of D-amino acid residues, the binding to D-biotin is low and the binding to L-biotin is high.

A core sequence of the polypeptide of the present invention may contain an L-amino acid residue, but it is preferable that 90% or more of amino acid residues among amino acid residues other than glycine are D-amino acid residues. In a more preferred embodiment, 95% or more of the amino acid residues among amino acid residues other than glycine in the core sequence are D-amino acid residues, and in a further preferred embodiment, all amino acid residues other than glycine are D-amino acid residues.

The polypeptide of the present invention may be a polypeptide containing an amino acid modification such as substitution, deletion or addition as compared to the above-described amino acid sequences. A preferred embodiment is a polypeptide having a homology of 90% or more with the above-described amino acid sequences, and a more preferred embodiment is a polypeptide having a homology of 95% or more with the above-described amino acid sequences.

Specific examples of the polypeptide with a modified amino acid sequence include a streptavidin variant described in Qureshi et al., THE JOURNAL OF BIOLOGICAL CHEMISTRY, Vol. 276, No. 49, Issue of December 7, pp. 46422-46428, 2001 (hereinafter, referred to as "streptavidin variant 1"). The streptavidin variant 1 has substitution mutations of S45A, T90A, and D128A (SEQ ID NO: 5) as compared with the amino acid sequence of SEQ ID NO: 1. The homology between the core sequence of streptavidin and a core sequence of streptavidin variant 1 is 97.4%. Streptavidin of one embodiment is D-form streptavidin variant 1 (hereinafter, referred to as "D-streptavidin variant 1"), which contains the 19th to 133rd amino acid sequence of SEQ ID NO: 5. The 19th to 133rd amino acid sequence is known as the core sequence of streptavidin variant 1. In a more preferred embodiment, the D-streptavidin variant 1 contains the 13th to 133rd amino acid sequence or the 19th to 139th amino acid sequence. In a further preferred embodiment, the D-streptavidin variant 1 contains the 13th to 139th amino acid sequence. In another embodiment, the D-streptavidin variant 1 contains an entire amino acid sequence of SEQ ID NO: 5.

Other examples of the streptavidin variant include a streptavidin variant described in Wu et al., THE JOURNAL OF BIOLOGICAL CHEMISTRY, Vol. 280, No. 24, Issue of June 17, pp.23225-23231, 2005 (hereinafter, referred to as "streptavidin variant 2"). The streptavidin variant 2 has substitution mutations of T76R, V125R, V55T, and L109T (SEQ ID NO: 6) as compared with the amino acid sequence of SEQ ID NO: 1. The homology between the core sequence of streptavidin and a core sequence of streptavidin variant 2 is 96.5%. Streptavidin of one embodiment is D-form streptavidin variant 2 (hereinafter, referred to as "D-streptavidin variant 2"), which contains the 19th to 133rd amino acid sequence of SEQ ID NO: 6. The 19th to 133rd amino acid sequence is known as the core sequence of streptavidin variant 2. In a more preferred embodiment, the D-streptavidin variant 2 contains the 13th to 133rd amino acid sequence or the 19th to 139th amino acid sequence. In a further preferred embodiment, the D-streptavidin variant 2 contains the 13th to 139th amino acid sequence. In another embodiment, the D-streptavidin variant 2 contains an entire amino acid sequence of SEQ ID NO: 6.

Other examples of the streptavidin variant include a streptavidin variant described in Lim et al., BIOTECHNOLOGY AND BIOENGINEERING, 2013 Jan; 110(1): 57-67 (hereinafter, referred to as "streptavidin variant 3"). This protein has the amino acid sequence of SEQ ID NO: 7. It is preferred that streptavidin of one embodiment is D-form streptavidin variant 3 (hereinafter, referred to as "D-streptavidin variant 3") and contains an entire amino acid sequence of SEQ ID NO: 7.

Other examples of the streptavidin variant include a variant described in Sano et al., Proc. Natl. Acad. Sci. USA Vol. 94, pp.6153-6158, June 1997 (hereinafter, referred to as "streptavidin variant 4"). The streptavidin variant 4 has a substitution mutation of H127D and a deletion mutation of G113 to W120 as compared with the amino acid sequence of SEQ ID NO: 1 (SEQ ID NO: 8). The homology between the core sequence of streptavidin and the core sequence of streptavidin variant 2 is 92.2%. Streptavidin of one embodiment is D-form streptavidin variant 4 (hereinafter, referred to as "D-streptavidin variant 4"), which contains the 19th to 125th amino acid sequence of SEQ ID NO: 8. The 19th to 125th amino acid sequence is known as a core sequence of streptavidin variant 4. In a more preferred embodiment, the D-streptavidin variant 4 contains the 13th to 125th amino acid sequence or the 19th to 131st amino acid sequence. In a further preferred embodiment, the D-streptavidin variant 4 contains the 13th to 131st amino acid sequence. In another embodiment, the D-streptavidin variant 4 contains an entire amino acid sequence of SEQ ID NO: 8.

Other examples of the streptavidin variant include a variant described in WO 2006/058226 A (hereinafter, referred to as "streptavidin variant 5"). The streptavidin variant 5 has an amino acid sequence of SEQ ID NO: 9. Streptavidin of one embodiment is D-form streptavidin variant 5 (hereinafter, referred to as "D-streptavidin variant 5"), which contains the 1st to 20th, 35th to 196th and 213rd to 261st amino acid sequences of SEQ ID NO: 9. The 1st to 20th, 35th to 196th, and 213th to 261st amino acid sequences are known as sequences corresponding to a core sequence of the streptavidin variant 5. In a more preferred embodiment, the D-streptavidin variant 5 contains the 1st to 24th, 29th to 202nd and 207th to 261st amino acid sequences. In another embodiment, the D-streptavidin variant 5 contains an entire amino acid sequence of SEQ ID NO: 9.

The notation of D- or L- in the present specification is a notation indicating the configuration of a compound based on IUPAC nomenclature, and is based on a notation in which a compound capable of not losing this configuration is denoted as D-form and an enantiomer thereof is denoted as L-form with d-glyceraldehyde as a reference for the configuration.

L-biotin, which is a binding partner of the polypeptide of the present invention, is an enantiomer of D-biotin. In the present specification, the "L-biotin" is a concept including free L-biotin and an L-biotin group added to other substances such as a capture body. L-biotin is synthesized by a known method such as the method of Non Patent Document (Journal of the American Chemical Society 1978, 100, 1558 to 1563), and can be obtained by optical resolution using chiral column chromatography or the like. The production method of L-biotin is not particularly limited as long as it is an enantiomer of D-biotin, and a commercially available product may be used.

The binding of the polypeptide of the present invention to L-biotin or D-biotin can be confirmed using, for example, a signal in a plate assay using a biotinylated enzyme and a polypeptide-immobilized plate, a change in a sensorgram in surface plasmon resonance (SPR), and a dissociation constant (Kd value) as indices. As a method for measuring the dissociation constant, for example, a known method using SPR analysis, isothermal titration type calorimetry analysis, or the like can be used.
As the measuring device, for example, Biacore T200 (Cytiva) is exemplified.

The dissociation constant of the polypeptide of the present invention and L-biotin is preferably 10⁻⁷ M or less, more preferably 10⁻¹⁰ M or less, and most preferably 10⁻¹³ M or less.

The phrase "having substantially no binding ability to D-biotin" refers that, for example, the dissociation constant of the polypeptide of the present invention and D-biotin is preferably 10⁻⁶ M or more, more preferably 10⁻⁴ M or more, and further preferably 10⁻² M or more. In a most preferred embodiment, it refers that when the binding ability between the polypeptide of the present invention and L-biotin is measured using Biacore T200 (Cytiva), a specific change in the sensorgram due to binding of analyte is not confirmed. Note that the phrase "having substantially no binding ability to D-biotin" is synonymous with "not substantially binding to D-biotin".

A method of determining the binding using a Biacore T200 system is, for example, as follows. Bovine serum albumin is immobilized to flow cells 1 and 3 of a CM5 sensor chip (manufactured by Cytiva) of the Biacore T200 system at a target level of 400 RU by an amine coupling method using an amine coupling kit (Cytiva).
Further, L-biotin-labeled albumin and D-biotin-labeled albumin are immobilized at a target level of 400 RU to flow cells 2 and 4, respectively, similarly by an amine coupling method. Each flow cell is blocked with 1 M ethanolamine solution, pH 8.5 (manufactured by Cytiva). As the D-biotin-labeled albumin, one estimated to have a labeling number of D-biotin per albumin by HABA method of 0.4 is used, and as the L-biotin-labeled albumin, one estimated to have a similar labeling number of D-biotin by being prepared under the same conditions as those of the D-biotin-labeled albumin is used.

Using HBS-EP+ (manufactured by Cytiva) as a running buffer, a polypeptide belonging to the avidin-streptavidin family is flowed into each flow cell at a flow rate of 30 µL/min in a range of 100 pM to 100 nM by a single cycle method, and data indicating a change in a sensorgram in SPR for D-biotin-labeled albumin or L-biotin-labeled albumin is acquired by Biacore T200 Evaluation Software (manufactured by Cytiva). Since the data acquired from the flow cell immobilized with bovine serum albumin shows background values, the background values may be used to normalize data showing changes in sensorgrams in SPR for D-biotin-labeled albumin or L-biotin-labeled albumin. Binding of the polypeptide belonging to the avidin-streptavidin family can be determined, for example, based on [data indicating binding between L-biotin-labeled albumin and polypeptide belonging to the avidin-streptavidin family] (hereinafter, "data L1") and [data indicating binding between D-biotin-labeled albumin and polypeptide belonging to the avidin-streptavidin family] (hereinafter, "data D 1").

For example, when the value obtained by dividing the value of data D1 by the value of data L1 is 1/10 or less, 1/100 or less, or 1/1000, the polypeptide belonging to the avidin-streptavidin family can be determined as "not substantially binding to D-biotin".

For example, when the value obtained by dividing the value of data L1 by the value of data D1 is 10 or more, 100 or more, or 1000 or more, the polypeptide belonging to the avidin-streptavidin family can be determined as "not substantially binding to D-biotin".

For example, when the absolute value of the value obtained by subtracting the value of data D1 from the value of data L1 or a value obtained by subtracting the value of data L1 from the value of data D1 is 1/10 or less, 1/100 or less, or 1/1000 of the value of data L1, the polypeptide belonging to the avidin-streptavidin family can be determined as "not substantially binding to D-biotin".

As a method for determining binding based on a signal in a plate assay using a biotinylated enzyme and a polypeptide-immobilized plate, for example, a biotin measurement system using an ELISA method is constructed based on Example 3 described later, and the value of enzyme activity when L-biotin is used (data L2) and the value of enzyme activity when D-biotin is used (data D2) are acquired. As with the case of using the Biacore T200 system, it is determined whether the polypeptide belonging to the avidin-streptavidin family substantially binds to D-biotin based on the data L2 and D2.

### 2. Multimer

Another related aspect of the invention is a multimer having the above-described polypeptide as a monomer unit. This multimer can be formed by association of a plurality of the above-described polypeptides. In this multimer, the number of monomer units is not particularly limited. For example, the multimer is a dimer, a tetramer, or an octamer.

### 3. Method for synthesizing polypeptide

The polypeptide of the present invention can be produced by a known method for synthesizing a peptide. The method for synthesizing a peptide is not particularly limited as long as the desired polypeptide is obtained. Examples thereof include liquid phase synthesis, solid phase synthesis, and synthesis by a cell-free system using artificial tRNA. When the number of amino acid residues of the polypeptide product is a certain number or more (generally 30 residues to 50 residues or more), a polypeptide can be produced by synthesizing two or more peptide fragments and then linking the polypeptides using a known ligation reaction.

The polypeptide of the present invention can be synthesized, for example, by the following solid phase synthesis method.
(1) A carboxyl group of a first amino acid residue protecting a nitrogen atom of an amino group is bound to a resin using a protecting group.
(2) The protecting group of the reactant obtained by the binding reaction of the above (1) or (3) is eliminated using a deprotecting agent, and then washed with a solvent to form a free amino acid.
(3) The free amino acid obtained in the above (2) is condensed with any amino acid having an amino group protected by the protecting group using a condensing agent.
(4) The protecting group of the product of the above (3) is eliminated using a deprotecting agent to form a free amino acid.
(5) By repeating the steps (2) to (4), it is possible to obtain a polypeptide to which an arbitrary amino acid to which a resin is bound is linked at the C-terminus.
(6) Washing is performed using a solvent at an arbitrary time point after the step (5) and at a time point when a resin to which a desired polypeptide is bound is produced.
(7) After the N-terminal amino group of the polypeptide to which the resin washed in the above (6) is bound is protected by a protecting group, the resin is cleaved using an acid, whereby any polypeptide to which the protecting group is bound can be obtained.

Each step may be manual synthesis, or an automated synthesizer or the like may be used. A part of the synthesis may be performed by manual synthesis, and an automated synthesizer may be appropriately used. Examples of the automated synthesizer include, but are not particularly limited to, Liberty Blue (manufactured by CEM Corporation), Multipep2 (manufactured by CEM Corporation), Initiator + Alstra (manufactured by Biotage), SyrII (manufactured by Biotage), and the like.

The resin used in (1) may be a known resin used in a solid phase synthesis method. As the resin that supplies the C-terminus as an amide group, for example, Rink-Amide-resin (manufactured by Merck KGaA), Rink-Amide-PEGA-resin (manufactured by Merck KGaA), and Fmoc-NH-SAL-resin (manufactured by WATANABE CHEMICAL INDUSTRIES, LTD.) functionalized with an amino group are preferably used. Fmoc-NH-SAL-resin-linker (manufactured by WATANABE CHEMICAL INDUSTRIES, LTD.) or the like may be bonded to Amino-PEGA-resin (manufactured by Merck KGaA) functionalized with an amino group or the like.

As the resin for forming a carboxylic acid at the C-terminus, for example, 2-chlorotrityl chloride resin functionalized with chlorine (manufactured by Merck KGaA), Amino-PEGA-resin functionalized with an amino group (manufactured by Merck KGaA), NovaSyn TGT alcohol resin having a hydroxyl group (manufactured by Merck KGaA), Wang-resin (manufactured by Merck KGaA), HMPA-PEGA-resin (manufactured by Merck KGaA), and the like can be used. A linker may be present between the Amino-PEGA-resin and the amino acid, and examples of such a linker include 4-hydroxymethylphenoxyacetic acid (HMPA), 4-(4-hydroxymethyl-3-methoxyphenoxy)-butylacetic acid (HMPB), and the like. H-Cys(Trt)-Trityl NovaPEG resin (manufactured by Merck KGaA) in which a C-terminal amino acid is previously bonded to a resin or the like can be used.

When a resin having a hydroxyl group or a resin functionalized with chlorine is used, a bond between the resin and an amino acid in which a nitrogen atom of an amino group is protected by a protecting group bonds a carboxyl group of the amino acid to the resin by an ester bond. When a resin functionalized with an amino group is used, the carboxyl group of the amino acid is bonded to the resin by an amide bond.

The protecting group may be any known protecting group, and for example, carbonate-based or amide-based protecting groups such as a 9-fluorenylmethoxycarbonyl (Fmoc) group, a t-butyloxylcarbonyl (Boc) group, a benzyl group, an allyloxycarbonyl group, and an acetyl group can be used. When a protecting group is introduced into an amino acid, for example, in the case of introducing an Fmoc group, the protecting group can be introduced by adding 9-fluorenylmethoxycarbonyl-N-succinimidyl carbonate and sodium carbonate and performing a reaction. The reaction temperature is 0 to 50°C, and preferably room temperature, and the reaction time is 1 to 5 hours, and preferably 3 hours.

As the amino acid in which the nitrogen atom of the amino group is protected using the protecting group, a commercially available amino acid may be used. Examples thereof include Fmoc-Ser-OH, Fmoc-Asn-OH, Fmoc-Val-OH, Fmoc-Leu-OH, Fmoc-Ile-OH, Fmoc-Ala-OH, Fmoc-Tyr-OH, Fmoc-Gly-OH, Fmoc-Lys-OH, Fmoc-Arg-OH, Fmoc-His-OH, Fmoc-Asp-OH, Fmoc-Glu-OH, Fmoc-Gln-OH, Fmoc-Thr-OH, Fmoc-Cys-OH, Fmoc-Met-OH, Fmoc-Phe-OH, Fmoc-Trp-OH, Fmoc-Pro-OH, Fmoc-SeMet-OH, and Fmoc-3-(Methylseleno)-Ala-OH.

An amino acid protected by a protecting group and having a protecting group introduced into a side chain may be used. Examples thereof include Fmoc-Arg(Pbf)-OH, Fmoc-Asn(Trt)-OH, Fmoc-Asp(tBu)-OH, Fmoc-Cys(Acm)-OH, Fmoc-Cys(tBu)-OH, Fmoc-Cys(tBu)-OH, Fmoc-Cys(Trt)-OH, Fmoc-Glu(tBu)-OH, Fmoc-Gln(Trt)-OH, Fmoc-His(Trt)-OH, Fmoc-Lys(Boc)-OH, Fmoc-Ser(tBu)-OH, Fmoc-Thr(tBu)-OH, Fmoc-Trp(Boc)-OH, Fmoc-Tyr(tBu)-OH, Fmoc-Sec(Trt)-OH, Fmoc-Sec(pMeOBzl)-OH, Fmoc-Sec(pMeBzl)-OH, Fmoc-HomoSec(pMeBzl)-OH, and Fmoc-HomoSec(Mob)-OH.

When a resin having a hydroxyl group is used, for example, an HMPB resin can be used as an esterification catalyst. At that time, as the dehydration condensing agent, a known dehydration condensing agent such as 1-mesitylenesulfonyl-3-nitro-1,2,4-triazole (MSNT), dicyclohexylcarbodiimide (DCC), or diisopropylcarbodiimide (DIC) can be used. As for the use ratio of the amino acid and the dehydration condensing agent, the latter is used in an amount of usually 1 to 10 equivalents and preferably 1 to 5 equivalents based on 1 equivalent of the former.

The esterification reaction is preferably carried out by, for example, subjecting a resin to a solid phase column, washing with a solvent, and adding an amino acid solution. Examples of the washing solvent include dimethylformamide (DMF), 2-propanol, dichloromethane (DCM), and the like. Examples of the solvent for dissolving an amino acid include dimethyl sulfoxide (DMSO), DMF, DCM, and the like. The reaction temperature of the esterification reaction is 0 to 50°C, and preferably room temperature, and the reaction time is about 10 minutes to 30 hours, and preferably about 15 minutes to 24 hours.

At this time, it is preferable to acetylate and cap the unreacted functional group on the solid phase using acetic anhydride or the like.

The lipid-soluble protecting group can be eliminated by, for example, a treatment with a base. Examples of the base include piperidine, morpholine, and the like. At that time, it is preferably performed in the presence of a solvent. Examples of the solvent include DMF, DMSO, methanol, and the like.

An amidation reaction between a free amino group and a carboxy group of any amino acid in which the amino group nitrogen is protected by the protecting group is preferably carried out in the presence of an activating agent, a base, and a solvent.

Examples of the activating agent include DIC, DCC, 1-ethyl-3-(3-dimethylaminopropyl)carbodiimide hydrochloride (WSC · HCl), diphenyl phosphoryl azide (DPPA), carbonyl diimidazole (CDI), diethyl cyanophosphonate (DEPC), benzotriazol-1-yloxy-trispyrrolidinophosphonium hexafluorophosphate (PyBOP), 1-hydroxybenzotriazole (HOBt), hydroxysuccinimide (HOSu), dimethylaminopyridine (DMAP), 1-hydroxy-7-azabenzotriazole (HOAt), hydroxyphthalimide (HOPht), pentafluorophenol (Pfp-OH), O-(1H-6-chlorobenzotriazol-1-yl)-1,1,3,3-tetramethyluronium hexafluorophosphate (HCTU), O-(7-azabenzotriazol-1-yl)-1,1,3,3-tetramethyluronium hexafluorophosphonate (HATU), 2-(1H-benzotriazol-1-yl)-1,1,3,3-tetramethyluronium tetrafluoroborate (TBTU), 3,4-dihydro-3-hydroxy-4-oxo-1,2,3-benzotriazine (DHBT), 4-(4,6-dimethoxy-1,3,5-triazin-2-yl)-4-methylmorpholinium chloride (DMT-MM), and the like.

The amount of the activating agent used is 1 to 20 equivalents, preferably 1 to 10 equivalents, and further preferably 1 to 5 equivalents with respect to any amino acid having an amino group nitrogen protected by a protecting group.

As the base, a base that can coexist with an alkylation reaction is preferable. Examples thereof include, but are not particularly limited to, N-ethyldiisopropylamine (DIPEA), 1,8-diazabicyclo[5.4.0]undec-7-ene (DBU), DMAP, 1,4-diazabicyclo[2.2.2]octane (DABCO), 2,6-dimethylpyridine, triethylamine (TEA), 1,5-diazabicyclo[4.3.0]nona-5-ene (DBN), and the like.

Examples of the solvent include DMF, DMSO, DCM, and the like. The reaction temperature is 0 to 50°C, and preferably room temperature, and the reaction time is about 10 minutes to 30 hours, and preferably about 15 minutes to 24 hours. The elimination of the protecting group can be performed in the same manner as described above.

In order to cleave the peptide chain from the resin, it is preferable to treat with an acid. Examples of the acid include trifluoroacetic acid (TFA).

As a known ligation reaction for linking two or more peptide fragments, native chemical ligation (NCL method) can be used (Dawson et al., Synthesis of Proteins by Native Chemical Ligation. Science, 266: 776-779 (1994)).

The NCL method is a chemoselective reaction between a first peptide having an α-carboxythioester moiety at the C-terminus and a second peptide having a cysteine residue at the N-terminus, a thiol group (also referred to as an SH group or a sulfhydryl group) of the cysteine side chain selectively reacts with carbonyl carbon of a thioester group, and a thioester binding initial intermediate is produced by a thiol exchange reaction. This intermediate spontaneously undergoes intramolecular rearrangement to give a native amide bond at the ligation site while regenerating a cysteine side-chain thiol.

In the NCL method, a cysteine binding site of the second peptide having a cysteine residue at the N-terminus can also be substituted with alanine by a desulfurization reaction (Yanet al., J. Am. Chem. Soc. 123, 526(2001)) after the ligation reaction. That is, a site that is originally alanine is substituted with cysteine to perform synthesis, and can be used as a binding site for the ligation reaction.

Steps before and after the peptide synthesis reaction or the ligation reaction may include a separation and/or purification step. As the purification method, a known method may be used, and examples thereof include column chromatography and the like. Examples of the column chromatography include, but are not particularly limited to, normal phase chromatography, reverse phase chromatography, gel filtration chromatography, affinity chromatography, and the like. A solvent, a filler of a column, a method for detecting an object to be separated and an object to be purified, a temperature condition, a pressure condition, and the like can be appropriately selected depending on the object to be separated and purified.

Known washing, drying, dilution, concentration steps, and the like may be appropriately included before and after the peptide synthesis reaction, ligation reaction, or separation and/or purification step.

When the polypeptide is not folded correctly, it is preferred to refold the polypeptide. Refolding can be performed by, for example, lysing the purified polypeptide in a denaturing buffer including urea, guanidine hydrochloride or the like or adding a denaturant such as urea or guanidine hydrochloride to the roughly purified solution of the fusion polypeptide to lyse aggregation of the fusion polypeptide, and then performing, for example, dilution refolding, dialysis refolding, solid phase refolding, size exclusion chromatography refolding, surfactant refolding, or the like, described in Non Patent Document (Arakawa et al., Antibodies 232 (2014)). Dialysis refolding is preferable.

### 4. Measurement method

The measurement method of the present invention is a method of measuring a test substance in a specimen in vitro using a solid phase on which the above-described polypeptide or multimer thereof is immobilized and a capture body immobilized on the solid phase.

As the capture body, a substance that specifically binds to the test substance can be used. The capture body is directly or indirectly added with L-biotin or a variant thereof. Examples of the capture body include, but are not particularly limited to, antibodies, antigens, aptamers, lectins, nucleic acids, enzymes, and the like.

The "antibody" used as the capture body or the detector described below may be a full-length antibody or a fragment thereof. The antibody class may be any of IgG, IgA, IgM, IgD and IgE, and is preferably IgG. A subclass of IgG is not particularly limited, and may be any of IgG1, IgG2, IgG3 and IgG4. Examples of the fragment of the antibody include reduced IgG (rIgG), Fab, Fab', F(ab'), F(ab')2, Fv, single chain antibody (scFv), diabody, triabody, and the like. Methods for preparing these antibody fragments are known per se. The antibody may be either a monoclonal antibody or a polyclonal antibody, but is preferably a monoclonal antibody. The monoclonal antibody may be a chimeric antibody, a humanized antibody, a fully humanized antibody, or the like. Also, the antibody may be an antibody derived from any animal such as a mouse, a rat, a hamster, a rabbit, a goat, a horse, a camel, an alpaca, or a chicken.

The test substance is a substance to be detected by the measurement method of the present invention, and may be any substance that can be captured by the capture body. Examples of the test substance include, but are not particularly limited to, cells, extracellular vesicles, proteins, nucleic acids, polysaccharides, glycoproteins, phospholipids, and the like.

The specimen is a sample containing a test substance or a sample suspected of containing a test substance. Examples of the specimen include samples derived from organisms, environmental samples, samples obtained by subjecting these samples to pretreatment, and the like. Examples of the sample derived from organisms include body fluids and excretions. Examples of the body fluid include, but are not particularly limited to, serum, plasma, blood, cerebrospinal fluid, semen, tissue, tissue fluid, lymph fluid, saliva, nasopharyngeal swab fluid, sputum, bronchoalveolar lavage fluid, and the like as long as the body fluid is a sample collected from a living body. Examples of the excretion include, but are not particularly limited to, urine, feces, and the like. Examples of the environmental sample include sewage, river water, seawater, soil, and the like.

The above-described polypeptide or multimer thereof is immobilized on a solid phase. The mode of immobilization is not particularly limited. For example, the above-described polypeptide or multimer thereof may be directly or indirectly bound to the solid phase. Examples of the direct binding include physical adsorption. The indirect binding is a mode in which another substance is interposed between the above-described polypeptide or multimer thereof and the solid phase. For example, when the surface of the solid phase is coated with a blocking agent such as bovine serum albumin or polyethylene glycol, the above-described polypeptide or multimer thereof can be immobilized on the solid phase by binding the above-described polypeptide or multimer thereof to the solid phase and the blocking agent.

In the measurement method of the present invention, the capture body and the solid phase may be separately provided to the user, or the capture body and the solid phase may be provided to the user in a state of being fixed to the solid phase in advance. When the capture body and the solid phase are separately provided to the user, the measurement method of the present invention may include a step of contacting the specimen, the solid phase, and the capture body. In the step, the order of contact between the specimen, the solid phase, and the capture body is not particularly limited. Preferably, the specimen and the capture body are contacted with each other to form a complex of the test substance in the specimen and the capture body, and then the complex and the solid phase are contacted with each other to form the complex on the solid phase. In this case, even if the specimen contains D-biotin, the polypeptide or multimer immobilized on the solid phase does not substantially bind to D-biotin and can bind to the capture body containing the L-biotin group.

In the measurement method of the present invention, it is preferable that the capture body and the solid phase are separately provided to the user in that the solid phase can be made common to a plurality of kinds of test substances.

The measurement method of the present invention may include the steps of: forming a complex containing a capture body, a test substance, and a detector (hereinafter, also referred to as "sandwich complex") on a solid phase (hereinafter, also referred to as a "complex forming step"); and measuring a signal based on the detector contained in the complex (hereinafter, also referred to as "measurement step").

In the complex forming step, a sandwich complex can be formed on the solid phase by contacting the solid phase, the capture body, the specimen containing the test substance, and the detector with each other. The order of contact between the solid phase, the capture body, the specimen, and the detector is not particularly limited. Preferably, the specimen and the capture body are contacted with each other to form a complex of the test substance in the specimen and the capture body, then the complex and the solid phase are contacted with each other to form a complex on the solid phase, and then the complex and the detector are contacted with each other to form a sandwich complex on the solid phase. In this case, even if the specimen contains D-biotin, the polypeptide or multimer immobilized on the solid phase does not substantially bind to D-biotin and can bind to the capture body containing the L-biotin group. After forming the complex of the test substance and the capture body on the solid phase, it is preferable to perform B/F separation for removing unreacted components before contacting with the detector. The reaction conditions (for example, solvent, temperature, pressure, reaction time, etc.) of each component in the complex forming step can be appropriately selected by those skilled in the art.

The detector preferably contains a substance that binds to the test substance and a labeling substance. Examples of the substance that binds to the test substance include antibodies, antigens, aptamers, lectins, nucleic acids, enzymes, and the like. In addition, the detector may contain a primary substance that binds to the test substance and a secondary substance that contains a labeling substance and binds to the primary substance. Examples of the primary substance and the secondary substance include antibodies, antigens, aptamers, lectins, nucleic acids, enzymes, and the like. When both the primary substance and the secondary substance are antibodies, they are referred to as a primary antibody and a secondary antibody, respectively, and are widely used in the art. When the primary antibody and the secondary antibody are used, a sandwich complex containing a labeling substance can be formed by binding the primary antibody to the test substance and binding the secondary antibody to the primary antibody. As a specific example, the primary substance is a primary antibody derived from an animal such as a mouse or a rabbit and specifically binding to the test substance, and the secondary substance is a secondary antibody containing a labeling substance and specifically binding to an antibody of the animal.

Examples of the labeling substance include, but are not particularly limited to, substances which themselves generate a signal (hereinafter also referred to as "signal generating substance"), and substances which catalyze the reaction of other substances to generate a signal. Examples of the signal generating substance include fluorescent substances, radioactive isotopes, and color developing substances. Examples of the substance which catalyzes a reaction of other substances to generate a detectable signal include enzymes. Examples of the fluorescent substances include fluorescent dyes such as fluorescein isothiocyanate (FITC), rhodamine and Alexa Fluor (registered trademark), and fluorescent proteins such as GFP. Examples of the radioactive isotopes include ¹²⁵I, ¹⁴C, and ³²P. Examples of the color developing substances include metal colloids such as gold nanocolloid. Examples of the enzyme include alkaline phosphatase, peroxidase, β-galactosidase, glucosidase, polyphenol oxidase, tyrosinase, acid phosphatase, and luciferase. A preferred labeling substance is an enzyme, and alkaline phosphatase is particularly preferred.

In the measurement step, the test substance can be measured by measuring a signal based on the detector. Based on the value obtained by the measurement step, determination, qualitative detection or semi-quantitative detection of the test substance can be performed. Here, the semi-quantitative detection means to show the signal intensity in stages like "no signal generated", "weak", "medium", "strong", and the like.

Methods for detecting a signal itself are known in the art. In the present invention, a method according to the type of signal derived from the labeling substance may be appropriately selected. As the signal detection device, for example, an absorption spectrometer, a spectrophotometer, a fluorometer, an infrared photometer, a Raman spectrophotometer, an SPR measurement device, a chemiluminescent enzyme immunoassay device, or the like can be used, but it is not particularly limited thereto.

In one embodiment, the test substance in the specimen is immunologically measured. Examples of the immunological measurement method include an ELISA method, an immunochromatography method, an immune complex transfer method described in JP-A-1-254868, and the like.

In another embodiment, a target nucleic acid in the specimen is measured as the test substance. For example, a solid phase on which the above-described polypeptide or multimer is immobilized and a nucleic acid probe that hybridizes to a target nucleic acid as a capture body can be used. The nucleic acid probe is labeled with L-biotin and binds to the polypeptide or multimer of the present invention on the solid phase. By contacting the solid phase, the target nucleic acid, and the specimen with each other, the target nucleic acid in the specimen is immobilized on the solid phase. On the solid phase, an extension reaction of DNA is performed by DNA polymerase and dNPTs using a target nucleic acid as a template and a nucleic acid probe as a primer. Detection of DNA can be performed by a known method. For example, the synthesized amplicon can be labeled with an intercalator (for example, SYBR (trademark) Green I dye, ethidium bromide, and the like) as a detector, and fluorescence can be measured. Alternatively, a fluorescent probe capable of binding to the elongated chain is bound as a detector, and fluorescence can be measured. In addition, TaqMan (trademark) method may be used.

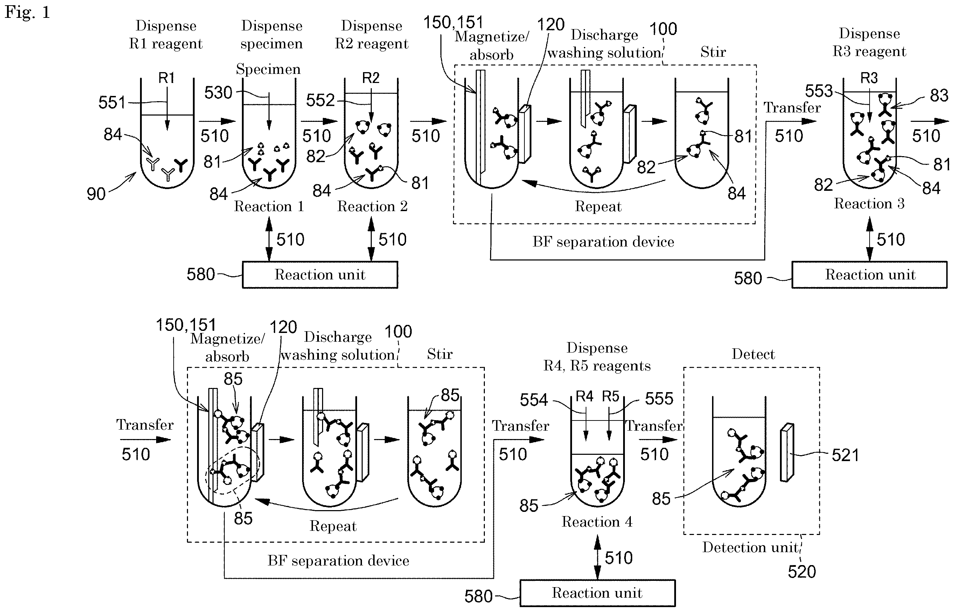

With reference to Fig. 1, an example of the measurement method of the present invention will be described.

First, a specimen containing a test substance 81 and an R1 reagent are dispensed into a container 90. The R1 reagent is dispensed into the container 90 by a first reagent dispenser 551, and the specimen is dispensed into the container 90 by a specimen dispenser 530. The R1 reagent contains a capture body 84 to which L-biotin is added, and reacts with and binds to the test substance 81. After dispensing the specimen and the R1 reagent, a sample in the container 90 is heated to a predetermined temperature in a reaction unit 580, whereby the capture body 84 and the test substance 81 are bound to each other.

Next, an R2 reagent is dispensed into the container 90 by a second reagent dispenser 552. The R2 reagent contains a solid phase 82. On the solid phase 82, the polypeptide of the present invention or a multimer thereof is immobilized. After dispensing the R2 reagent, the sample in the container 90 is heated to a predetermined temperature in the reaction unit 580. As a result, the test substance 81 and the capture body 84 are immobilized on the solid phase 82 by binding the L-biotin to the polypeptide of the present invention or a multimer thereof on the solid phase.

The test substance 81 and the capture body 84 formed on the solid phase 82 and an unreacted capture body 84 may be separated by primary BF separation treatment by a BF separation device 100. By the primary BF separation treatment, unnecessary components such as the unreacted capture body 84 are removed from the container 90.

Next, an R3 reagent is dispensed into the container 90 by a third reagent dispenser 553. The R3 reagent contains a detector 83, and reacts with and binds to the test substance 81. After dispensing the R3 reagent, the sample in the container 90 is heated to a predetermined temperature in the reaction unit 580. As a result, a sandwich complex 85 containing the test substance 81, the detector 83, and the capture body 84 is formed on the solid phase 82. In the example of Fig. 1, the detector 83 is an enzyme-labeled antibody.

The sandwich complex 85 formed on the solid phase 82 and an unreacted labeling substance 83 are separated by secondary BF separation treatment by the BF separation device 100. By the secondary BF separation process, unnecessary components such as the unreacted detector 83 are removed from the container 90.

Thereafter, an R4 reagent and an R5 reagent are dispensed into the container 90 by each of a fourth reagent dispenser 554 and a fifth reagent dispenser 555. The R4 reagent contains a buffer solution. The sandwich complex 85 bound to the solid phase 82 is dispersed in a buffer solution. In the example of Fig. 1, the R5 reagent contains a chemiluminescent substrate. The buffer solution contained in the R4 reagent has a composition that promotes a reaction between the enzyme labeled in the detector 83 contained in the sandwich complex 85 and the chemiluminescent substrate contained in the R5 reagent. After dispensing the R4 and R5 reagents, the sample in the container 90 is heated to a predetermined temperature in the reaction unit 580. Light is generated by reacting the substrate with the detector 83, and the generated light is detected by a photodetector 521 of the detection unit 520. The test substance 81 is measured based on the intensity of the detected light.

In this measurement method, the R1 reagent and the R2 reagent are separately dispensed into the container 90, but the R1 reagent and the R2 reagent may be mixed in advance and dispensed into the container 90 in a state where the capture body 84 is immobilized on the solid phase 82.

### 5. Solid phase and reagent kit including solid phase

The solid phase of the present invention is a solid phase on which the above-described polypeptide or multimer thereof is immobilized.

The solid phase of the present invention is an insoluble carrier for immobilizing the capture body. The mode of immobilization of the above-described polypeptide or multimer thereof on the solid phase is as described above. The solid phase can be selected from known solid phases according to the purpose. The solid phase material can be selected from, for example, polymer compounds, inorganic substances, and the like. As the solid phase material, a plurality of materials may be appropriately combined.

The polymer compound includes an organic polymer compound, an inorganic polymer compound, and a semi-organic compound. Examples of the polymer compound include latex, rubber, polystyrene, polyethylene, polypropylene, a styrene-butadiene copolymer, polyvinyl chloride, polyvinyl acetate, polyacrylamide, polymethacrylate, a styrene-methacrylate copolymer, polyglycidyl methacrylate, an acrolein-ethylene glycol dimethacrylate copolymer, polyvinylidene difluoride (PVDF), silicone, insoluble agarose, insoluble dextran, and the like.

The inorganic substance includes an inorganic compound and a metal. Examples of the inorganic compound include magnetic bodies (iron oxide, chromium oxide, cobalt, nickel, ferrite, magnetite, and the like), glass, silica, alumina, and the like. Examples of the metal include gold, silver, and substances containing them.

The reagent kit of the present invention includes a solid phase to which the above-described polypeptide or multimer thereof is bound, and a capture body to which L-biotin is added.

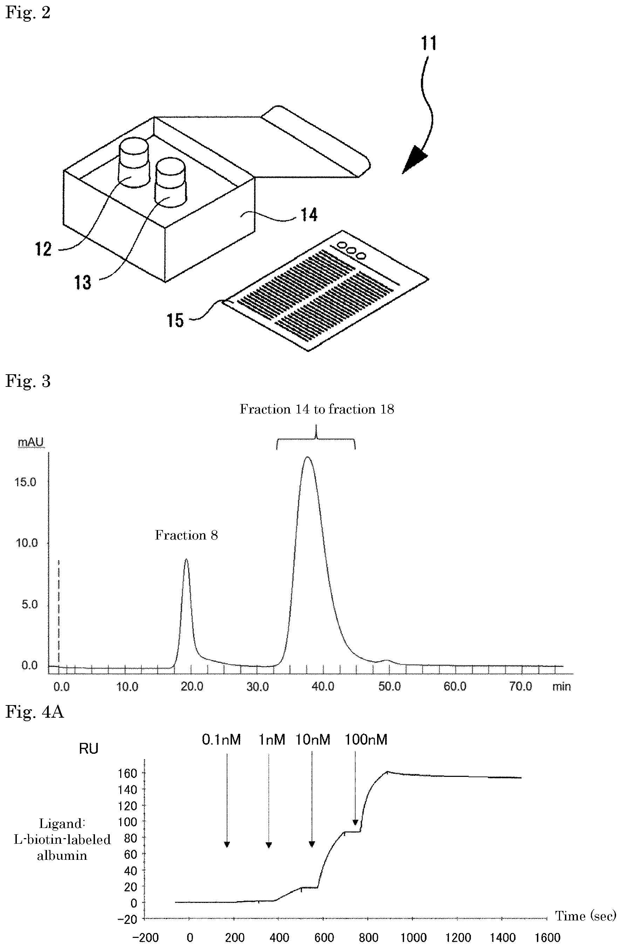

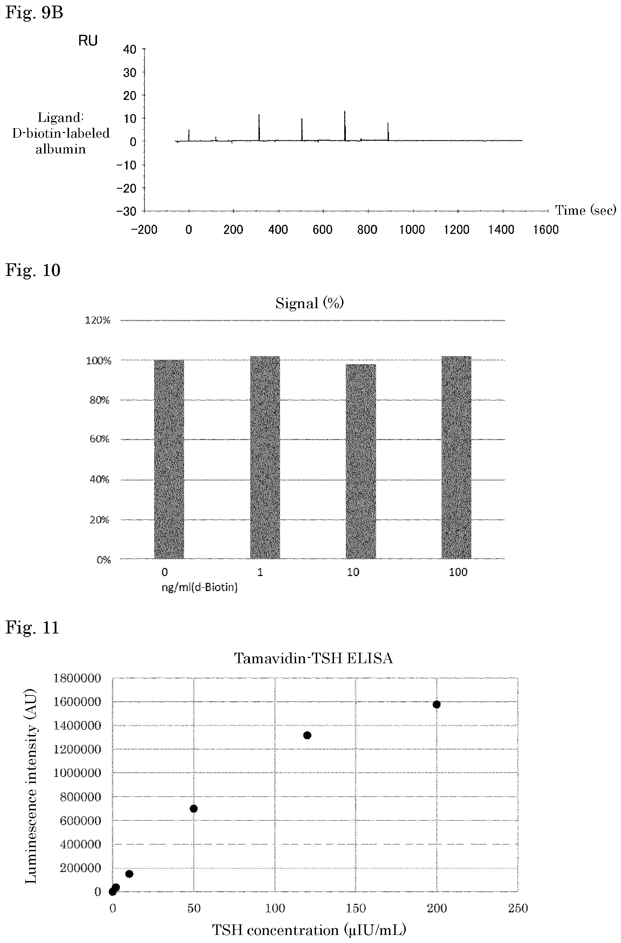

An example of the reagent kit of the present invention is shown in Fig. 2. In Fig. 2, 11 denotes a reagent kit, 12 denotes a first container storing a reagent containing particles to which the above-described polypeptide or multimer thereof is bound, 13 denotes a second container storing a reagent containing a capture body to which L-biotin is added, 14 denotes a packing box, and 15 denotes an attached document. Composition, usage, storage method, etc. of each reagent may be described in the attached document. The reagent kit may include other reagents, and examples thereof include a buffer solution, a calibrator, a detector, and the like.

### EXAMPLES

The present invention will be described below in more detail, with reference to examples. However, the present invention is not construed as limited to the examples.

### 1. Example 1: Preparation of D-Streptavidin

### 1-1. Method

In order to synthesize a polypeptide composed of the 13th to 139th amino acids of a polypeptide set forth in SEQ ID NO: 1 (D-core streptavidin), a peptide thioester composed of the 13th to 71st amino acids of SEQ ID NO: 1, and a polypeptide composed of the 139th amino acid of the polypeptide in which the 72nd alanine residue was substituted with cysteine were synthesized.
(1) DCM was added to HMPB-ChemMatrix resin (0.49 mmol/g, manufactured by Biotage) (820 mg, 400 µmol), and the mixture was swollen at room temperature for 1 hour or more. Next, Fmoc-D-Thr(tBu)-OH (795 mg, 2.0 mmol) which is an amino acid having a side chain protected by a protecting group, MSNT (593 mg, 2.0 mmol) as a condensing agent, and 1-methylimidazole (160 µL, 2.0 mmol) as a capping agent for a 5'-terminal hydroxyl group were added to DCM (12 mL), and the mixture was mixed. The mixed solution was added to the swollen resin and stirred at room temperature for 3 hours.
(2) After removing the reaction solution, the resin was sufficiently washed with DCM and DMF. Next, acetic anhydride (2.0 mL, 20 mmol) and pyridine (1.6 mL, 20 mmol) were added to DMF (12 mL), and the mixture was well mixed, mixed with the resin, and stirred at room temperature. After 30 minutes, the solution was removed and the resin sufficiently washed with DMF.
(3) To a resin (100 µmol) obtained by condensing Fmoc-D-Thr(tBu)-OH, Fmoc-Gly-OH (500 µmol), DIC (500 µmol) as an activating agent, Oxyma Pure (trademark) (500 µmol) (manufactured by Merck KGaA) as an anti-epimerization agent, and N-methylpyrrolidone (NMP) (4 mL) as a solvent were added, and the mixture was condensed. In the same procedure, the amino acid having a side chain protected by a protecting group was changed to Fmoc-D-Ser(tBu)-OH (500µmol), Fmoc-D-Asp(OtBu)-(Hmb)-Gly-OH (first: 150 µmol, second: 100 µmol), and condensation was performed by manual synthesis. Fmoc-D-Asp(OtBu)-(Hmb)-Gly-OH was condensed by double coupling.
(4) 28 Residues at 39L to 66T were extended using a microwave-assisted automated peptide synthesizer (LibertyBlue, manufactured by CEM Corporation). In the microwave-assisted automated peptide synthesizer, Fmoc-D-amino acid (500 µmol), DIC (500 µmol), Oxyma Pure (trademark) (500 µmol) and DIPEA (50 µmol) were used, DMF was used for the condensation reaction and washing of the resin, and 20% piperidine/DMF was used for removing an Fmoc group. At the time of the condensation reaction and the removal reaction of the Fmoc group, the reaction was promoted by irradiation with a microwave.
(5) The resin was recovered, and peptides of Fmoc-D-Ala-OH and Fmoc-D-Asp(OtBu)-(Hmb)-Gly-OH were extended again by manual synthesis under the same reaction conditions as in (3). Here, double coupling of Fmoc-D-Asp(OtBu)-(Hmb)-Gly-OH was performed twice at 100 µmol. Acetic anhydride (167 µL, 1.8 mmol), DIPEA (78.8 µL, 0.45 mmol) and Oxyma Pure (trademark) (5.3 mg, 37 µmol) were added to NMP (7 mL) to the resin-bound polypeptide, mixed, and stirred at room temperature for 15 minutes, then sufficiently washed with NMP.
(6) 23 Residues at 13A to 35A were extended using a microwave-assisted automated peptide synthesizer to finally prepare 59 residue polypeptide at 13A to 71T to which the resin was bound.
(7) The 59 residue polypeptide to which the resin was bound was recovered from the synthesizer in a state in which the N-terminal Fmoc group was removed. Boc₂O (115 µL, 500 µmol), DIPEA (87 µL, 500 µmol) and NMP (3 mL) were added to the recovered resin (100 µmol), and the mixture was stirred at room temperature.
   After 1 hour, the solution was removed and the resin was sufficiently washed with NMP. This Bocylation operation was repeated twice.
(8) A 1% TFA/DCM solution (3 mL) was added to the 59 residue polypeptide to which the resin was bound (100 µmol), and the mixture was stirred for 5 minutes. Then, the filtrate was collected in a recovery flask containing pyridine (585 µL). This operation was repeated 13 times, then the resin was washed 3 times with a trifluoroethanol/DCM (3: 5) solution (3 mL), and the washing liquid was collected in the same recovery flask. The solvent was distilled off under reduced pressure, then H₂O (40 mL) was added to the obtained solid, and the mixture was stirred. The mixture was centrifuged for 10 minutes (10,000 rpm) to remove the supernatant. This operation was repeated three times, and the individual was washed, then the obtained solid was freeze-dried to obtain a protected peptide (171 mg, 20 µmol).
(9) The resulting protected peptide (116 mg, 13.5 µmol) was dissolved in NMP (5 mL) and cooled to -15°C with stirring. Thiophenol (42 µL, 405 µmol), PyBOP (36.7 mg, 67.5 µmol), and DIPEA (12 µL, 67.5 µmol) were added thereto, and the mixture was stirred overnight. After completion of the reaction, the reaction solution was poured into ice-cooled diethyl ether (40 mL) and stirred. The mixture was centrifuged for 10 minutes (10,000 rpm) to remove the supernatant. This operation was repeated three times, and then the resulting precipitate was air-dried to obtain a protected crude peptide thioester (thiophenyl ester form) (113 mg). A mixed liquid (3 mL) of TFA: H₂O: triisopropylsilane (TIPS): ethanedithiol (EDT) (92.5: 2.5: 2.5: 2.5, v/v/v/v) was added to the obtained crude product, and the mixture was stirred at room temperature. After 3 hours, the reaction solution was added to ice-cooled diethyl ether (40 mL), and the resulting precipitate was air-dried to obtain a deprotected crude product peptide (85.6 mg). To the obtained deprotected crude peptide, a 0.2 M phosphate buffer (pH 7.0) (3 mL) containing 8 M guanidine hydrochloride and 0.2 M sodium 2-mercaptoethanesulfonate (MESNA) was added, and the mixture was stirred at room temperature. The mixture was stirred for 1 hour or more, and then purified by RP-HPLC (Proteonavi C1, 4.6 × 250 mm, Linear gradient of B: 17.5-21.5, 1.0 mL/min, 12 min, 60°C, 220 nm, A: aqueous solution containing 0.1% TFA, B: 90% MeCN, 10% H₂O containing 0.09% TFA, manufactured by Shiseido Company, Limited) to obtain a 59 residue peptide thioester (13A to 71T, MESNA thioester form) (6.6 mg, 1.09 µmol).

### (Synthesis of polypeptide containing cysteine)

(1) DCM was added to HMPB-ChemMatrix resin (0.49 mmol/g) (612 mg, 300 µmol), and the mixture was swollen at room temperature for 1 hour or more. Next, Fmoc-D-Ser(tBu)-OH (575 mg, 1.5 mmol), MSNT (444 mg, 1.5 mmol) and 1-methylimidazole (120 µL, 1.5 mmol) were added to DCM (9 mL), and the mixture was well mixed. The mixed solution was added to the swollen resin and stirred at room temperature overnight. The reaction solution was removed, and the resin was washed sufficiently with DCM and DMF. Next, acetic anhydride (1.5 mL, 15 mmol) and pyridine (1.2 mL, 15 mmol) were added to DMF (9 mL), and the mixture was mixed with the resin and stirred at room temperature for 30 minutes. Thereafter, the resin was sufficiently washed with DMF.
(2) Using a microwave-assisted automated peptide synthesizer, a resin (100 µmol) obtained by condensing Fmoc-D-Ser(tBu)-OH was extended 68 residues at 72C to 139S. The amounts of the reagents used in the condensation reaction were Fmoc-D-amino acid (500 µmol), DIC (500 µmol), Oxyma Pure (trademark) (500 µmol), and DIPEA (50 µmol). DMF was used for the condensation reaction and the washing of the resin, and 20% piperidine/DMF was used for the removal of the Fmoc group. In the condensation and the removal reaction of the Fmoc group, the reaction was promoted by irradiation with a microwave.
(3) To the 68 residue polypeptide to which the resin was bound (100 µmol), a mixed solution of TFA: H₂O: TIPS: EDT (92.5: 2.5: 2.5: 2.5, v/v/v/v) (10 mL) was added, and the mixture was stirred at room temperature. After 3 hours, the reaction solution was added to ice-cooled diethyl ether (80 mL), and the resulting precipitate was air-dried to obtain a deprotected crude product peptide (396 mg).
(4) The obtained deprotected crude peptide was purified by RP-HPLC (Triart-C18, 20 × 250 mm, Linear gradient of B: 30-45, 9.9 mL/min, 30 min, 60°C, 220 nm, A: aqueous solution containing 0.1% TFA, B: 90% MeCN, 10% H₂O containing 0.09% TFA, manufactured by YMC CO., LTD) to obtain a 68 residue peptide segment (72C to 139S) (37.2 mg, 5.03 µmol).

### (Ligation of polypeptide)

Peptide thioester (13A to 71T) (6.0 mg, 0.933 µmol) and peptide segment (72C to 139S) (9.3 mg, 1.26 µmol) were added to a 0.2 M phosphate buffer (containing 8 M guanidine hydrochloride, 40 mM 4-mercaptoacetic acid, 40 mM sodium ascorbate, and 40 mM tris(2-carboxyethyl)phosphine (TCEP), pH 7.1) (546 µL), and the mixture was stirred at room temperature through the night. After completion of the reaction, the mixture was diluted 2 times with a 0.2 M phosphate buffer (containing 8 M guanidine hydrochloride and 0.2 M sodium 2-mercaptoethanesulfonate, pH 7.0), and stirred at room temperature. After stirring for 1 hour or more, the diluted mixed solution was purified by RP-HPLC (Proteonavi C1, 4.6 × 250 mm, Linear gradient of B: 15-35, 1.0 mL/min, 30 min, 60°C, 220 nm, A: aqueous solution containing 0.1% TFA, B: 90% MeCN, 10% H₂O containing 0.09% TFA, manufactured by Shiseido Company, Limited) to obtain a full-length 127 residue [Cys60]-(1-127) peptide (6.7 mg, 0.504 µmol).

### (Desulfurization reaction)

The [Cys60]-(1-127) peptide (4.7 mg, 0.354 µmol) was dissolved in 707 µL of a 0.2 M phosphate buffer (pH 7.1) containing 8 M guanidine hydrochloride and 0.25 M TCEP, and tert-butanethiol (40 µL) and VA-044/H₂O (250 mg/mL, 27 µL) were sequentially added, then the container was shielded from light, and the mixture was stirred at room temperature. After 4 hours, 1.4 mL of the 0.2 M phosphate buffer (pH 7.1) containing 8 M guanidine hydrochloride and 0.25 M TCEP were added to the mixture and mixed well. Diethyl ether (1.7 mL) was added to this mixed solution, and the resulting mixture was stirred well and then allowed to stand to recover an aqueous phase. This procedure was repeated three times to sufficiently remove diethyl ether from the recovered aqueous phase, and then the resulting mixture was purified by RP-HPLC (Proteonavi C1, 4.6 × 250 mm, Linear gradient of B: 15-45, 1.0 mL/min, 30 min, 60°C, 220 nm, A: aqueous solution containing 0.1% TFA, B: 90% MeCN, 10% H₂O containing 0.09% TFA, manufactured by Shiseido Company, Limited) to obtain a full-length 127 residue polypeptide (2.6 mg, 0.196 µmol).

### (Preparation of tetramer)

The full-length 127 residue polypeptide was dissolved in a denaturation buffer (50 mM Tris-HCl, 6 M guanidine-HCl, 1 mM EDTA, 200 mM NaCl, pH 8.0 (4°C)) so as to be 1 mg/mL, and the solution was heated at 85°C for 45 minutes. 5 mL of the heated solution was dialyzed in buffer 1 (50 mM Tris-HCl, 1 mM EDTA, 200 mM NaCl, pH 8.0) containing 3 M guanidine HCl at 4°C for 12 hours. Next, dialysis was performed against buffer 1 containing 2 M guanidine HCl at 4°C for 36 hours.
Dialysis was performed against buffer 1 containing 0.4 M L-arginine HCl and 1 M guanidine HCl at 4°C for 12 hours. Dialysis was performed against buffer 1 containing 0.4 M L-arginine HCl and 0.5 M guanidine HCl at 4°C for 36 hours.
Finally, dialysis was repeated twice against buffer 1 at 4°C for 4 hours, and dialysis was performed against buffer 1 at room temperature overnight.

### (Gel filtration chromatography analysis)

The dialyzed polypeptide was concentrated with an Amicon Ultra-15 10K centrifugal filter device (manufactured by Merck KGaA), then subjected to Superdex 200 10/300GL (manufactured by Cytiva) in an amount of 500 µL each using an AKTA Prime system (manufactured by Cytiva) and flowing buffer 1 at a flow rate of 0.4 mL/min to perform gel filtration chromatography using a temporal change in absorbance at a wavelength of 280 nm as an index of elutability. Further, for estimating the molecular weight, gel filtration chromatography was similarly performed for commercially available tetrameric natural core streptavidin (manufactured by Roche).

### 1-2. Results

A temporal change in absorbance at a wavelength of 280 nm of gel filtration chromatography of the polypeptide prepared above is shown in Fig. 3. Fraction 8 is a peak of aggregate. Fraction 14 to fraction 18 are fractions in which a similar peak was observed even in commercially available tetrameric core streptavidin, and thus are estimated to be a peak of the tetrameric polypeptide. Fraction 14 to fraction 18 were collected and concentrated using an Amicon Ultra-15 10K centrifugal filter device (manufactured by Merck KGaA) to prepare tetrameric D-streptavidin (hereinafter, for convenience, it is simply referred to as "D-streptavidin" in Examples) composed of D-amino acid residues.

### 2. Example 2: Biotin Binding Evaluation of D-Streptavidin by SPR Analysis

### 2-1. Method

A binding ability of D-streptavidin obtained in Example 1 to D-biotin as a natural form and L-biotin as an optical isomer of D-biotin was evaluated by an intermolecular interaction analyzer (Biacore T200) using SPR as a measurement principle.

L-Biotin was prepared by synthesizing a mixture of D-biotin and L-biotin by the method described in Non Patent Document (Journal of the American Chemical Society 1978, 100, 1558-1563), and then optical resolution by liquid chromatography was entrusted to Daicel Corporation. CHIRALPAK IG (Φ 46 × 50 mm, manufactured by Daicel Corporation) was used as a column, and optical resolution was performed using a methanol: acetic acid mixed solvent (100: 0.1 (v/v)) as a mobile phase under conditions of a flow rate of 1.0 mL/min, a column temperature of 40°C, and a detection wavelength of 205 nm.

### (1) Preparation of biotin-labeled albumin

An N,N-dimethylformamide solution (16.3 µL) containing 10 mg/mL D-biotin-AC5-OSu (manufactured by DOJINDO LABORATORIES) was added to 2.8 mL of 50 mg/mL bovine serum albumin/0.1 M phosphate buffer solution (pH 7.5), and the mixture was stirred, then allowed to stand at 35°C for 1 hour. Thereafter, desalting was performed by PD-10 (17085101, manufactured by Cytiva) equilibrated with a 0.1 M phosphate buffer (pH 7.5), and D-biotin-labeled albumin was recovered. The number of labeled D-biotin per molecule of albumin by HABA method was 0.4.

Similarly, L-biotin-labeled albumin was prepared using L-biotin-AC5-OSu [see (Amidation of L-biotin) described in Example 3 above].

### (2) Fixing to sensor chip

Bovine serum albumin was immobilized on flow cells 1 and 3 of a CM5 sensor chip (manufactured by Cytiva) of a Biacore T200 system at a target level of 400 RU by an amine coupling method with an amine coupling kit (Cytiva). Further, L-biotin-labeled albumin and D-biotin-labeled albumin were immobilized at a target level of 400 RU to flow cells 2 and 4, respectively, similarly by an amine coupling method. Blocking was performed in each flow cell with 1 M ethanolamine solution, pH 8.5 (manufactured by Cytiva).

### (Measurement of interaction with biotin)

HBS-EP+ (manufactured by Cytiva) was used as a running buffer, and D-streptavidin prepared in Example 1 as an analyte was flowed into each flow cell at a flow rate of 30 µL/min in a range of 10 pM to 100 nM by a single cycle method.

### 2-2. Results

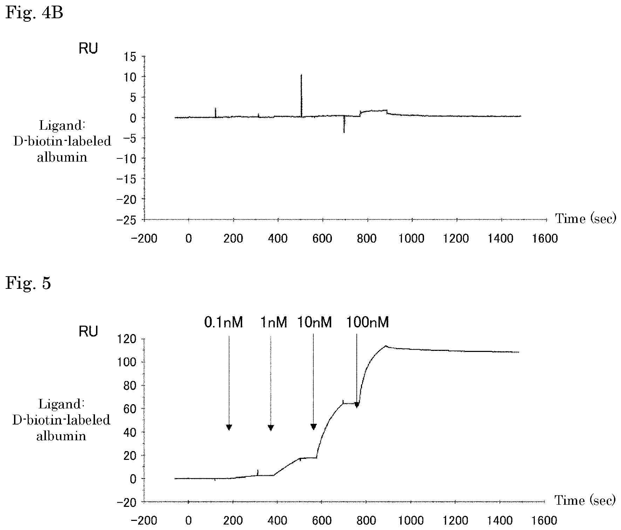

A sensorgram of the intermolecular interaction between D-streptavidin and L-biotin-labeled albumin is shown in Fig. 4A, and a sensorgram of the intermolecular interaction between D-streptavidin and D-biotin-labeled albumin is shown in Fig. 4B. A sensorgram of the intermolecular interaction between natural core streptavidin and D-biotin-labeled albumin is shown in Fig. 5.

From the comparison between the sensorgram of natural core streptavidin and D-biotin-labeled albumin and the sensorgram of D-streptavidin and L-biotin-labeled albumin, it was found that the D-streptavidin and the L-biotin-labeled albumin of this example were very strongly bound similarly to the combination of natural core streptavidin and D-biotin-labeled albumin.

In Fig. 4, a specific change in the sensorgram due to binding of D-streptavidin as an analyte between D-streptavidin and D-biotin-labeled albumin was not confirmed. Considering the dissociation constant Kd = 3.48 × 10⁻⁷ between the streptavidin mutant and the natural D-biotin-labeled albumin shown in Patent Document 1, it can be said that the binding between the D-streptavidin of this example and D-biotin is extremely low, and they are not substantially bound.

### 3. Example 3: Evaluation of Influence on Measurement System of D-Biotin in ELISA Method

### 3-1. Method

### (Production of streptavidin-bound solid phase)

A 96 well ELISA plate (manufactured by Thermo Fisher Scientific Inc.) was washed with PBS three times, then the D-streptavidin (1 µg/mL, PBS, 100 µL) prepared in Example 1 was added thereto, and the plate was allowed to stand still at 4°C overnight. After washing three times with PBS, a PBS buffer (200 µL) containing 2% bovine serum albumin was added, and the mixture was shaken and stirred at 25°C and a rotation speed of 600 rpm for 2 hours to prepare a D-streptavidin-immobilized plate. L-streptavidin (manufactured by Roche) was also subjected to the same treatment to prepare an L-streptavidin-immobilized plate.

### (Amidation of L-biotin)

Biotin (43 mg) and N-hydroxysuccinimide (NHS) (24.3 mg) were dissolved in DMF (1.2 mL), ethyl(dimethylaminopropyl)carboxydiimide (EDC) (40.5 mg) was added thereto, and the mixture was stirred at room temperature for 23 hours. The residue obtained by distilling off the solvent was recrystallized from ethanol: acetic acid: water (95: 5: 1) to obtain 6-aminohexanoic acid 6-(5-((3aR,4R,6aS)-2-oxohexahydro-1H-thieno[3,4-d]imidazol-4-yl)pentanamide)hexan oic acid (Compound 2) (63.9 mg).

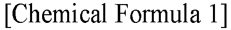

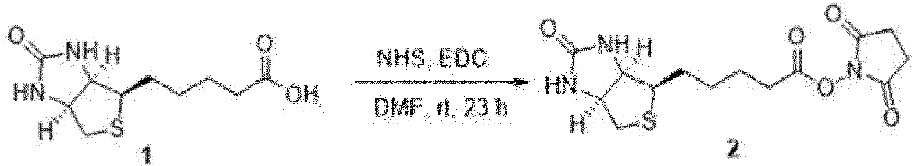

6-Aminohexanoic acid (24.2 mg)) was dissolved in a 0.25 M aqueous sodium carbonate solution (0.4 mL). This solution was added to a DMF (1 mL) solution of Compound 2, and the mixture was stirred at room temperature for 23 hours. After the solvent was distilled off, the residue was washed with water. The residue was acidified with 4 M hydrochloric acid, and the generated solid was collected by filtration to obtain 6-(5-((3 aR,4R,6aS)-2-oxohexahydro-1H-thieno[3,4-d]imidazol-4-yl)pentanamide)hexan oic acid (Compound 3) (68.5mg).

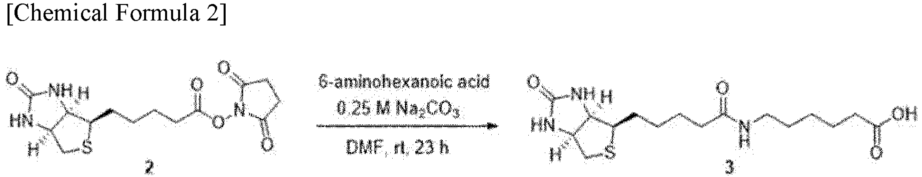

Compound 3 (68.5 mg) and NHS (40.5 mg) were dissolved in DMF (3.3 mL), and EDC (67.5 mg) was added at room temperature, then the mixture was stirred at 35°C for 21 hours. The residue obtained by distilling off the solvent was applied to a silica gel column chromatography, and 2,5-dioxopyrrolidin-1-yl 5-((3aR,4R,6aS)-2-oxohexahydro-1H-thieno[3,4-d]imidazol-4-yl)pentanoate (Compound 4) (47.6 mg) was obtained from a DCM-methanol elution part.

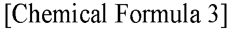

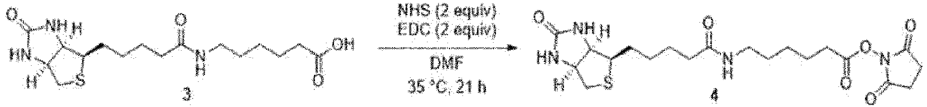

### (Preparation of biotin-labeled antibody)

A DMSO solution (1.3 µL) containing 16.7 mg/mL L-biotin-AC5-OSu was added to 5.1 mg/mL thyroid stimulating hormone antibody (TSH antibody) (T2-194) (manufactured by KITAYAMA LABES CO., LTD.) (25 µL), and the mixture was stirred, and then allowed to stand at 35°C for 1 hour. Thereafter, the sample was subjected to PD-10 (17085101, manufactured by Cytiva) equilibrated with a 0.1 M phosphate buffer (pH 7.5), fractions of 500 µL each were fractionated, and the absorption peak fraction at a wavelength of 280 nm was collected to prepare an L-biotin-labeled TSH antibody. The same treatment was also applied to D-biotin-AC5-OSu (manufactured by DOJINDO LABORATORIES) to produce a D-biotin-labeled TSH antibody.

### (Evaluation of influence on measurement system in presence of D-biotin)

(1) 400 µL of HISCL (trademark) TSH calibrator C3 (manufactured by Sysmex Corporation) as a specimen and 400 µL of HISCL (trademark) TSH R1 reagent (manufactured by Sysmex Corporation) were added to 1.5 mL tube, and reacted under conditions of 37°C and 600 rpm for 30 minutes. For the specimens, in order to evaluate the degree of influence of D-biotin, 0 ng/mL, 1 ng/mL, 10 ng/mL or 100 ng/mL of D-biotin was added and reacted with the R1 reagent.
(2) The D-streptavidin-immobilized plate after blocking was washed three times with a HISCL (trademark) wash solution (manufactured by Sysmex Corporation), and 60 µL of the solution reacted with 1.5 mL tubes was added to each well. The mixture was reacted at 37°C and 600 rpm for 20 minutes. The L-biotin-labeled TSH antibody prepared above was added, and the mixture was reacted under conditions of 37°C and 600 rpm for 30 minutes.
(3) After the reaction, the plate was washed five times with a HISCL (trademark) wash solution (manufactured by Sysmex Corporation), 50 µL each of a HISCL (trademark) R4 reagent (manufactured by Sysmex Corporation) was added, subsequently 100 µL each of a HISCL (trademark) R5 reagent (manufactured by Sysmex Corporation) was added, and the mixture was reacted under conditions of 37°C and 600 rpm for 30 minutes. After the reaction, luminescence detection was performed with a microplate reader (Infinite 200 PRO, manufactured by TECAN). The L-streptavidin-immobilized plate and the D-biotin-labeled TSH antibody after blocking were also measured in the same manner.

### 3-2. Results

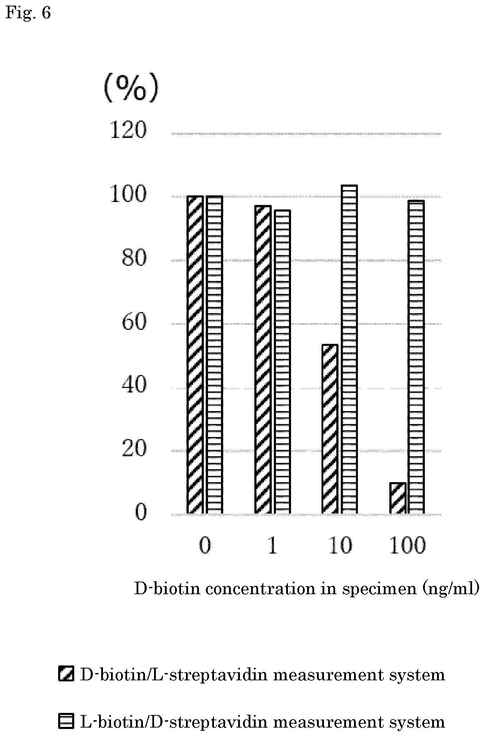

The detection result of TSH by the ELISA method in the presence of D-biotin is shown in Fig. 6. Fig. 6 shows percentages of signal measured values of each specimen when the signal measured value when measuring the specimen with a D-biotin concentration of 0 ng/mL in each measurement system is taken as 100%. When D-biotin was present in the specimen, the signal decreased depending on the addition concentration of D-biotin in the measurement system of D-biotin/L-streptavidin. On the other hand, in the measurement system of L-biotin/D-streptavidin, the effect due to the addition of D-biotin was hardly observed.

The measurement system of D-streptavidin/L-biotin was not substantially affected by endogenous D-biotin present in the specimen.

### 4. Example 4: Confirmation of Quantitability of Measurement System

### 4-1. Method

(1) HISCL (trademark) TSH Calibrators C0 to C5 (manufactured by Sysmex Corporation) were added as a specimen to a 1.5 mL tube so as to be 0 µIU/mL, 2 µIU/mL, 10 µIU/mL, 50 µIU/mL, 120 µIU/mL, or 200 µIU/mL. Thereafter, 400 µL of a HISCL (trademark) TSH R1 reagent (manufactured by Sysmex Corporation) added with HISCL (trademark) TSH calibrators C0 to C5 (manufactured by Sysmex Corporation) was added thereto, and the mixture was reacted under conditions of 37°C and 600 rpm for 30 minutes.
(2) The D-streptavidin-immobilized plate after blocking prepared in the same manner as in Example 3 was washed three times with a HISCL (trademark) wash solution (manufactured by Sysmex Corporation), and 60 µL each of the solutions reacted with 1.5 mL tubes were added to each well. The mixture was reacted at 37°C and 600 rpm for 20 minutes. The L-biotin-labeled TSH antibody prepared above was added, and the mixture was reacted under conditions of 37°C and 600 rpm for 30 minutes.
(3) After the reaction, the plate was washed five times with a HISCL (trademark) wash solution (manufactured by Sysmex Corporation), 50 µL each of a HISCL (trademark) R4 reagent (manufactured by Sysmex Corporation) was added, subsequently 100 µL each of a HISCL (trademark) R5 reagent (manufactured by Sysmex Corporation) was added, and the mixture was reacted under conditions of 37°C and 600 rpm for 30 minutes. After the reaction, luminescence detection was performed with a microplate reader (Infinite 200 PRO).

### 4-2. Results

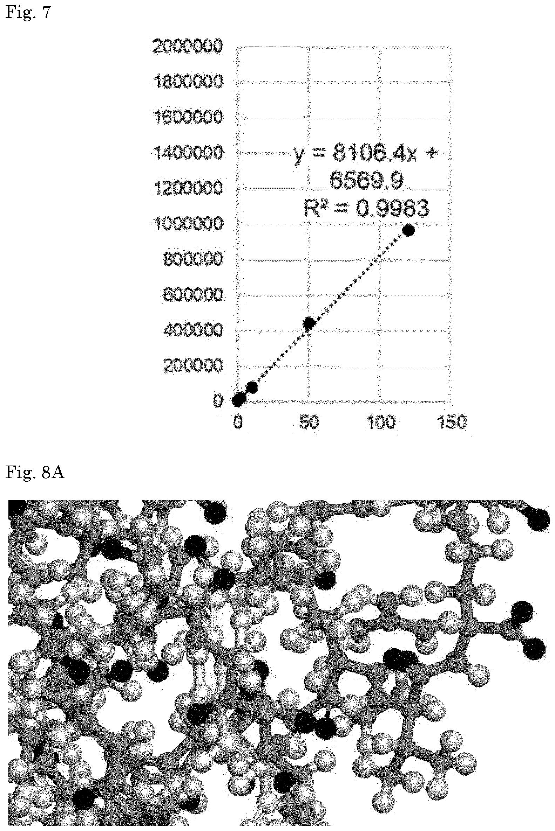

The results measured in Example 4 are shown in Table 1 and Fig. 7. Fig. 7 is a graph showing count values at TSH concentrations of 0 to 120 µIU/mL. As shown in Fig. 7, the L-biotin/D-streptavidin system showed a good correlation with R² = 0.998. Since the reference standard range of TSH was 0.34 to 4.22 µIU/mL, it was found to be a system having good linearity in the measurement range.

**[Table 1]**

| TSH Concentration (µIU/mL) | Calibrator | Count value |
|---|---|---|
| 0 | C0 | 1,010 |
| 2 | C1 | 17,272 |
| 10 | C2 | 80,448 |
| 50 | C3 | 442,005 |
| 120 | C4 | 967,485 |
| 200 | C5 | 1,264,387 |

In addition, five samples with a TSH concentration of 50 µIU/mL were prepared, and the TSH concentration was measured for each sample. The results are shown in Table 2. The coefficient of variation was about 5%. It was found that the measurement system of this example has no problem also in terms of reproducibility.

**[Table 2]**

| | Count value |
|---|---|
| Average | 409652.4 |
| Standard deviation | 20773.11 |
| Coefficient of variation | 5.07 |

### 5. Example 5: L-Amino Acid Residue Substitution of Amino Acid Residue Related to Structure Maintenance

### 5-1. Method

The influence on the steric structure by changing some amino acid residues in the amino acid sequence of D-streptavidin shown in SEQ ID NO: 1 to L-form was examined. In the examination, in silico analysis was performed by Discovery Studio (manufactured by Dassault Systemes).

First, a crystal structure (PDB ID: 3RY2) of a natural core streptavidin tetramer was obtained from the Protein Data Bank (PDB), and one monomer structure was extracted from the tetramer. Based on the extracted structure, all amino acid residues were converted to D-amino acid residues on Discovery Studio to construct a monomer structure of D-streptavidin. Thereafter, the degree of solvent contact of the amino acid residues constituting the D-streptavidin monomer structure was confirmed. Among the amino acid residues of the core sequence of D-streptavidin, amino acid residues other than glycine and having a degree of solvent contact of 25% or less were identified. As a result, the amino acid residues were total of 36 of 21st, 29th, 77th, 104th, 130th, 54th, 56th, 39th, 43rd, 27th, 75th, 31st, 92nd, 90th, 128th, 102nd, 33rd, 79th, 71st, 73th, 106th, 23rd, 60th, 96th, 81st, 122nd, 28th, 86th, 88th, 50th, 45th, 108th, 38th, 110th, 132nd, and 42nd amino acid residues of SEQ ID NO: 1 in the ascending order of the degree of solvent contact. These amino acid residues are considered to be particularly involved in maintaining the structure within the monomer of D-streptavidin. When one of the above amino acid residues was substituted with an L-amino acid residue, the structure of the compound was optimized using Clean Geometry function with respect to the substituted amino acid residue, the amino acid residue adjacent to the N-side of the substituted amino acid residue, and the amino acid residue adjacent to the C-side thereof. Thereafter, the presence or absence of collision between atoms inside D-streptavidin was confirmed from the steric structure.

### 5-2. Results

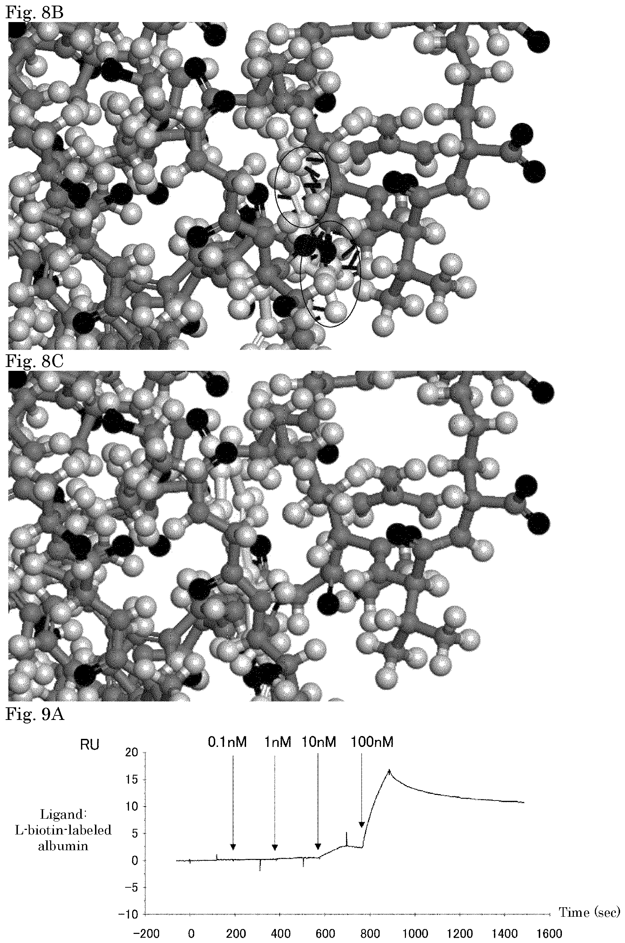

As a result of Example 5, for any amino acid residue of the 36 amino acid residues, the collision between atoms generated when the D-amino acid residue was substituted with the L-amino acid residue was resolved by performing structure optimization using the Clean Geometry function. As an example, Figs. 8A to 8C show a state in which the collision of atoms generated when the D-tryptophan residue as the 21st amino acid residue is substituted with an L-tryptophan residue is resolved. The structure before the substitution of the 21st D-tryptophan residue with the L-form is shown in Fig. 8A. Fig. 8B shows a state in which the collision between atoms occurs in D-streptavidin when the 21st D-tryptophan residue is substituted with the L-tryptophan residue (a cylinder highlighted by an ellipse represents the collision between atoms). Fig. 8C shows a state in which the collision between atoms in D-streptavidin is resolved by performing structure optimization using the Clean Geometry function.

In silico analysis, it was found that for any amino acid residue among amino acid residues involved in structure maintenance, the collision between atoms generated when the D-amino acid residue is substituted with the L-amino acid residue is resolved. Even in the in vitro state, it is considered that there is a structure in which an atom in D-streptavidin moves to resolve the collision between atoms when a part of amino acid residues involved in structure maintenance is substituted with the L-amino acid residue.

Therefore, it is considered that the structure capable of binding to biotin is maintained even if some of the amino acid residues involved in structure maintenance of the monomer are changed to L-amino acid residues.

### 6. Example 6: Synthesis of Avidin-like Protein Derived from Pleurotus cornucopiae Composed of D Amino Acids

An avidin-like protein (hereinafter, D-tamavidin (trademark) 2) derived from Pleurotus cornucopiae consisting of D amino acids was synthesized. The polypeptide having the 2nd to 141st amino acid sequence of SEQ ID NO: 4 was divided into five peptide segments, and each of the peptide segments was synthesized by an automated peptide synthesizer (Prelude, Protein Technologies, Inc.). The five segments were a segment 1 including the 2nd to 23rd sequence of SEQ ID NO: 4, a segment 2 including the 24th to 49th sequence, a segment 3 including the 50th to 76th sequence, a segment 4 including the 77th to 105th sequence, and a segment 5 including the 106th to 141st sequence. Next, the segments 1 and 2 were linked by chemical ligation to prepare a segment 1-2, the segments 3 and 4 were linked by chemical ligation to prepare a segment 3-4, the segment 5 was linked to the segment 3-4 by chemical ligation to prepare segment 3-4-5, and the segment 1-2 and the segment 3-4-5 were linked by chemical ligation to produce D-tamavidin (trademark) 2 having the 2nd to 141st amino acid sequence of SEQ ID NO: 4.

### (Preparation of tetramer)

D-Tamavidin (trademark) 2 was dissolved in a denaturation buffer (80 mM Tris-HCl, 6 M guanidine-HCl, 1 mM DTT, pH 8.0) so as to be 20 mg/mL, and the solution was heated at 85°C for 45 minutes. A buffer (80 mM Tris-HCl, 1 mM DTT, pH 8.0) was added in an amount of 5 times to the heated solution, and the solution was allowed to stand at room temperature for 30 minutes. Next, the solution was diluted 50 times with a buffer (80 mM Tris-HCl, 1 mM DTT, pH 8.0), and then allowed to stand O/N at 4°C. Thereafter, the solution was concentrated with Amicon Ultra-4 (30 k) (Merck Millipore), and then collected as a D-tamavidin (trademark) 2 protein solution.

### 7. Example 7: Biotin Binding Evaluation of D-Tamavidin (trademark) 2 by SPR Analysis

### 7-1. Method

### (1) Preparation of biotin-labeled albumin

An N,N-dimethylformamide solution (16.3 µL) containing 10 mg/mL D-biotin-AC5-OSu (manufactured by DOJINDO LABORATORIES) was added to 2.8 mL of 50 mg/mL bovine serum albumin/0.1 M phosphate buffer solution (pH 7.5), and the mixture was stirred, then allowed to stand at 35°C for 1 hour. Thereafter, desalting was performed by PD-10 (17085101, manufactured by Cytiva) equilibrated with a 0.1 M phosphate buffer (pH 7.5), and D-biotin-labeled albumin was recovered. The number of labeled D-biotin per molecule of albumin by HABA method was 0.4.

Similarly, L-biotin-labeled albumin was prepared using L-biotin-AC5-OSu [see (Amidation of L-biotin) described in Example 3 above].

### (2) Fixing to sensor chip

Bovine serum albumin was immobilized on flow cells 1 and 3 of a CM5 sensor chip (manufactured by Cytiva) of a Biacore T200 system at a target level of 400 RU by an amine coupling method with an amine coupling kit (Cytiva). Further, L-biotin-labeled albumin and D-biotin-labeled albumin were immobilized at a target level of 400 RU to flow cells 2 and 4, respectively, similarly by an amine coupling method. Blocking was performed in each flow cell with 1 M ethanolamine solution, pH 8.5 (manufactured by Cytiva).

### (3) Measurement of interaction with biotin

Using HBS-EP+ (manufactured by Cytiva) as a running buffer, the prepared D-tamavidin (trademark) 2 was flowed into each flow cell at a flow rate of 30 µL/min in the range of 100 pM to 100 nM by a single cycle method, and binding and dissociation to D-biotin or L-biotin was evaluated by Biacore T200 Evaluation Software (manufactured by Cytiva).

### 7-2. Results

The change in SPR signal of the intermolecular interaction between D-tamavidin (trademark) 2 and L-biotin-labeled albumin is shown in Fig. 9A. An increase in the SPR signal was observed depending on the addition amount of D-tamavidin (trademark) 2.

The SPR signal change of the intermolecular interaction between D-tamavidin (trademark) 2 and D-biotin-labeled albumin is shown in Fig. 9B. No increase in SPR signal dependent on the addition of D-tamavidin (trademark) 2 was observed.

These results showed that D-tamavidin (trademark) 2 does not substantially bind to D-biotin-labeled albumin but binds to L-biotin-labeled albumin.

### 8. Example 8: Evaluation of Influence on Measurement System of D-Biotin in ELISA Method Using D-Tamavidin (trademark) 2-Immobilized Plate

### 8-1. Method

(1) 200 µL of HISCL (trademark) TSH calibrator C3 (manufactured by Sysmex Corporation) as a specimen and 200 µL of HISCL (trademark) TSH R1 reagent (manufactured by Sysmex Corporation) were added to 1.5 mL tube, and reacted under conditions of 37°C and 600 rpm for 30 minutes. For the specimens, in order to evaluate the degree of influence of D-biotin, 0 ng/mL, 1 ng/mL, 10 ng/mL or 100 ng/mL of D-biotin was added and reacted with the R1 reagent.
   D-Tamavidin (trademark) 2 (1 µg/mL, PBS, 50 µL) was immobilized on a 96 well ELISA plate (Thermo Fisher Scientific Inc.) by allowing it to stand at 4°C overnight, and blocking was performed with 2% BSA/PBS.
(2) The D-tamavidin (trademark) 2-immobilized plate after blocking prepared in (1) was washed three times with a HISCL (trademark) wash solution (manufactured by Sysmex Corporation), and 60 µL of the solution reacted with 1.5 mL tubes was added to each well. The mixture was reacted at 37°C and 600 rpm for 20 minutes. The L-biotin-labeled TSH antibody prepared with reference to (Preparation of biotin-labeled antibody) described in Example 3 was added, and the mixture was reacted under conditions of 37°C and 600 rpm for 30 minutes.
(3) After the reaction, the plate was washed five times with a HISCL (trademark) wash solution (manufactured by Sysmex Corporation), 50 µL each of a HISCL (trademark) R4 reagent (manufactured by Sysmex Corporation) was added, subsequently 100 µL each of a HISCL (trademark) R5 reagent (manufactured by Sysmex Corporation) was added, and the mixture was reacted under conditions of 37°C and 600 rpm for 30 minutes. After the reaction, luminescence detection was performed with a microplate reader (SpectraMAXiD 3 Molecular Devices, LLC).

### 8-2. Results

The detection result of TSH by the ELISA method in the presence of D-biotin is shown in Fig. 10. Fig. 10 shows percentages of signal measured values of each specimen when the signal measured value when measuring the specimen with a D-biotin concentration of 0 ng/mL in each measurement system is taken as 100%. In the measurement system of L-biotin/D-tamavidin (trademark) 2, the effect due to the addition of D-biotin was hardly observed.

It was suggested that the measurement system of D-tamavidin (trademark) 2/L-biotin was not substantially affected by endogenous D-biotin present in the specimen.

### 9. Example 9: Confirmation of Quantitability of Measurement System Using D-tamavidin (trademark) 2-Immobilized Plate

### 9-1. Method

(1) 200 µL of HISCL (trademark) TSH Calibrators C0 to C5 (manufactured by Sysmex Corporation) were added as a specimen to a 1.5 mL tube so as to be 0 µIU/mL, 2 µIU/mL, 10 µIU/mL,50 µIU/mL, 120 µIU/mL,or 200 µIU/mL. Thereafter, 200 µL of a HISCL (trademark) TSH R1 reagent (manufactured by Sysmex Corporation) added with HISCL (trademark) TSH calibrators C0 to C5 (manufactured by Sysmex Corporation) was added thereto, and the mixture was reacted under conditions of 37°C and 600 rpm for 30 minutes.
(2) The D-tamavidin (trademark) 2-immobilized plate after blocking prepared in Example 8(1) was washed three times with a HISCL (trademark) wash solution (manufactured by Sysmex Corporation), and 60 µL of the solution reacted with 1.5 mL tubes was added to each well. The mixture was reacted at 37°C and 600 rpm for 20 minutes. The L-biotin-labeled TSH antibody prepared above was added, and the mixture was reacted under conditions of 37°C and 600 rpm for 30 minutes.
(3) After the reaction, the plate was washed five times with a HISCL (trademark) wash solution (manufactured by Sysmex Corporation), 50 µL each of a HISCL (trademark) R4 reagent (manufactured by Sysmex Corporation) was added, subsequently 100 µL each of a HISCL (trademark) R5 reagent (manufactured by Sysmex Corporation) was added, and the mixture was reacted under conditions of 37°C and 600 rpm for 30 minutes. After the reaction, luminescence detection was performed with a microplate reader (SpectraMAXiD 3 Molecular Devices, LLC).

### 9-2. Results

The measured results are shown in Table 3 and Fig. 11. A concentration-dependent signal change was observed.

**[Table 3]**

| TSH Concentration (µIU/mL) | Calibrator | Count value |
|---|---|---|
| 0 | C0 | 363 |
| 2 | C1 | 35344 |
| 10 | C2 | 149809 |
| 50 | C3 | 699577 |
| 120 | C4 | 1315798 |
| 200 | C5 | 1576566 |

In addition, five samples with a TSH concentration of 50 µIU/mL were prepared, and the TSH concentration was measured for each sample. The results are shown in Table 4. The coefficient of variation was about 5%. It was revealed that the measurement system of this example has no problem also in terms of reproducibility.

**[Table 4]**

| | Count value |
|---|---|
| Average | 667878.6 |
| Standard deviation | 32615. 67 |
| Coefficient of variation | 4. 88 |

### 10. Example 10: In Silico Analysis

Binding to L-biotin or D-biotin for each of D-bradavidin and D-avidin was studied by in silico analysis.

### 10-1. Method

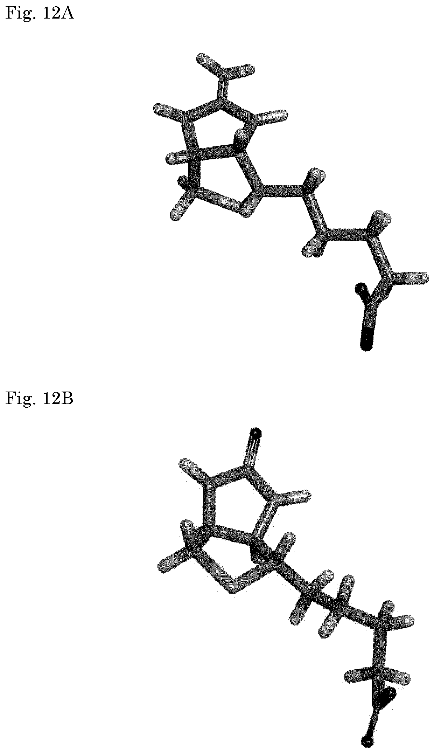

The structure of L-biotin used for the analysis is shown in Fig. 12A, and the structure of D-biotin is shown in Fig. 12B. Discovery Studio 2018 was used in preparation of the structure of L-biotin or D-biotin and calculation. In the analysis, a ligand other than D-biotin was removed in the form of a tetramer from the crystal structure data of the natural streptavidin-D-biotin complex (PDB ID: 3RY2 Biological Assembly 1), the crystal structure data of the natural bradavidin-D-biotin complex (PDB ID: 4BBO Biological Assembly 1), or the crystal structure data of the natural avidin-D-biotin complex (PDB ID: 2AVI Biological Assembly 1). Thereafter, a hydrogen atom was imparted using Prepare protein command to remove D-biotin from the structure. For each structure, a structure of an optical isomer protein composed of D-amino acid was generated by an LID Conversion command, and a binding site with a radius of 12 Å was set at a coordinate corresponding to the biotin binding site in the natural body, and used for ligand docking prediction of D-biotin and L-biotin.

The ligand docking structure was predicted by CDOCKER function on Discovery Studio, and the numerical value of interaction score (CDOCKER ENERGY) was obtained. The interaction score between the natural streptavidin and the optical isomer of each biotin is an index for binding.

### 10-2. Results

| The results were as follows. Each unit is kcal/mol | |
|---|---|
| a. D-Streptavidin | |
| L-Biotin | -33.5867 |
| D-Biotin | -29.2717 |
| b. D-Bradavidin | |
| L-Biotin | -30.6423 |
| D-Biotin | -25.9508 |
| c. D-Avidin | |
| L-Biotin | -31.5848 |
| D-Biotin | -27.6185 |

In all optical isomers, the score indicating the binding to L-biotin was lower by about 3.9 to 4.7 kcal/mol as compared with the score indicating the binding to D-biotin. This result suggests that D-bradavidin and D-avidin do not substantially bind to D-biotin and strongly bind to L-biotin, similarly to D-streptavidin.

## Claims

1. A polypeptide belonging to the avidin-streptavidin family, wherein
the polypeptide comprises a D-amino acid residue in 90% or more of amino acid residues among amino acid residues other than glycine of the polypeptide, has a binding ability to L-biotin, and has substantially no binding ability to D-biotin.

2. The polypeptide according to claim 1, comprising:
an amino acid sequence having a homology of 90% or more with any of the amino acid sequences of:
the 19th to 133rd sequence of the amino acid sequence set forth in SEQ ID NO: 1;
the 2nd to 128th sequence of the amino acid sequence set forth in SEQ ID NO: 2; the 4th to 129th sequence of the amino acid sequence set forth in SEQ ID NO: 3, and
the 4th to 127th sequence of the amino acid sequence set forth in SEQ ID NO: 4.

3. A polypeptide belonging to the avidin-streptavidin family, wherein amino acid residues other than glycine of the polypeptide are comprised only of D-amino acid residues.

4. The polypeptide according to claim 3, comprising:
any of the amino acid sequences of:
the 19th to 133rd sequence of the amino acid sequence set forth in SEQ ID NO: 1;
the 2nd to 128th sequence of the amino acid sequence set forth in SEQ ID NO: 2;
the 4th to 129th sequence of the amino acid sequence set forth in SEQ ID NO: 3, and
the 4th to 127th sequence of the amino acid sequence set forth in SEQ ID NO: 4.

5. The polypeptide according to any one of claims 1 to 4, comprising:
the 19th to 133rd amino acid residues of the amino acid sequence set forth in SEQ ID NO: 1.

6. The polypeptide according to any one of claims 1 to 5, comprising:
the 13th to 139th amino acid residues of the amino acid sequence set forth in SEQ ID NO: 1.

7. A multimer comprising the polypeptide according to any one of claims 1 to 6 as a monomer unit.

8. The multimer according to claim 7, wherein the multimer is a tetramer.

9. A solid phase on which the polypeptide according to any one of claims 1 to 6 and/or the multimer according to claim 7 or 8 is immobilized.

10. A measurement method for a test substance using a solid phase on which a polypeptide and/or a multimer is immobilized and a capture body that binds to the test substance in a specimen,
wherein the polypeptide belongs to the avidin-streptavidin family, comprises a D-amino acid residue in 90% or more of amino acid residues among amino acid residues other than glycine, has a binding ability to L-biotin, and has substantially no binding ability to D-biotin,
wherein the multimer is a multimer having the polypeptide as a monomer unit,
and
wherein the capture body is added with L-biotin, and the capture body is immobilized on the solid phase when the test substance is detected by binding the L-biotin to the polypeptide or the multimer on the solid phase.

11. The measurement method according to claim 10, wherein the capture body comprises an antibody, an antigen, a lectin, a nucleic acid, an enzyme, or a substrate.

12. The measurement method according to claim 10 or 11, comprising:
contacting the solid phase, the capture body, a detector that binds to the test substance, and the test substance with each other to form a complex containing the solid phase, the capture body, the detector, and the test substance; and
measuring the test substance based on the detector contained in the complex.

13. The method according to claim 12, wherein the detector comprises a fluorescent substance or an enzyme.

14. A reagent kit for measuring a test substance, comprising:
a solid phase on which a polypeptide and/or a multimer is immobilized and a capture body to which L-biotin is added,
wherein the polypeptide belongs to the avidin-streptavidin family, comprises a D-amino acid residue in 90% or more of amino acid residues among amino acid residues other than glycine, has a binding ability to L-biotin, and has substantially no binding ability to D-biotin, and
wherein the multimer is a multimer having the polypeptide as a monomer unit.

15. The reagent kit according to claim 14, which is used in the measurement method according to any one of claims 10 to 13.
